# EUROPEAN PATENT SPECIFICATION

(11) **EP 2 146 735 B1**
(45) Date of publication and mention of the grant of the patent: **20.06.2012**
(21) Application number: 08745748.7
(22) Date of filing: 14.04.2008
(51) Int. Cl.: A61K 38/17, A61K 31/337, A61K 45/06, A61P 35/00

(54) **COMPOSITIONS COMPRISING SPARC POLYPEPTIDES**
ZUSAMMENSETZUNGEN MIT SPARC POLYPEPTIDEN
COMPOSITIONS COMPRENANT DES POLYPEPTIDES SPARC

(30) Priority: 13.04.2007 US 923340 P
(43) Date of publication of application: 27.01.2010
(73) Proprietor: Abraxis BioScience, Inc., Los Angeles, CA 90025 (US)
(72) Inventor: TRIEU, Vuong, Calabasas, California 91302 (US); DESAI, Neil P., Los Angeles, California 90025 (US)
(74) Representative: Grünecker, Kinkeldey, Stockmair & Schwanhäusser
(86) International application number: PCT/US2008/060213
(87) International publication number: WO 2008/128169

(56) References cited:
- WO-A-2004/064785
- WO-A-2006/058425
- WO-A-2008/000079
- TAI ISABELLA T ET AL: "Genome-wide expression analysis of therapy-resistant tumors reveals SPARC as a novel target for cancer therapy." THE JOURNAL OF CLINICAL INVESTIGATION JUN 2005, vol. 115, no. 6, June 2005 (2005-06), pages 1492-1502, XP002488747 ISSN: 0021-9738
- DESAI N ET AL: "SPARC expression in breast tumors may correlate to increased tumor distribution of nanoparticle albumin-bound paclitaxel (ABI-007) vs taxol" BREAST CANCER RESEARCH AND TREATMENT, vol. 88, no. Suppl. 1, 2004, pages S26-S27, XP002488837 & 27TH ANNUAL CHARLES A COLTMAN SAN ANTONIO BREAST CANCER SYMPOSIUM; SAN ANTONIO, TX, USA; DECEMBER 08 -11, 2004 ISSN: 0167-6806
- GRADISHAR WILLIAM J: "Albumin-bound paclitaxel: a next-generation taxane." EXPERT OPINION ON PHARMACOTHERAPY JUN 2006, vol. 7, no. 8, June 2006 (2006-06), pages 1041-1053, XP009103437 ISSN: 1744-7666

### SEQUENCE LISTING

<110> ABRAXIS BIOSCIENCE, INC.
   Trieu, Vuong
   Desai, Neil P.
<120> SPARC AND METHODS OF USE THEREOF
<130> 702783
<140> PCTUS0860213
   <141> 2008-04-14
<150> US 60/923,340
   <151> 2007-04-13
<160> 4
<170> Patent In version 3.4
<210> 1
   <211> 286
   <212> PRT
   <213> Homo sapiens
<400> 1
   Ala Pro Gln Gln Glu Ala Leu Pro Asp Glu Thr Glu Val Val Glu Glu
   1               5                   10                  15
   Thr Val Ala Glu Val Thr Glu Val Ser Val Gly Ala Asn Pro Val Gln
               20                  25                  30
   Val Glu Val Gly Glu Phe Asp Asp Gly Ala Glu Glu Thr Glu Glu Glu
           35                  40                  45
   Val Val Ala Glu Asn Pro Cys Gln Asn His His Cys Lys His Gly Lys
       50                  55                  60
   Val Cys Glu Leu Asp Glu Asn Asn Thr Pro Met Cys Val Cys Gln Asp
   65                  70                  75                  80
   Pro Thr Ser Cys Pro Ala Pro Ile Gly Glu Phe Glu Lys Val Cys Ser
                   85                  90                  95
   Asn Asp Asn Lys Thr Phe Asp Ser Ser Cys His Phe Phe Ala Thr Lys
               100                 105                 110
   Cys Thr Leu Glu Gly Thr Lys Lys Gly His Lys Leu His Leu Asp Tyr
           115                 120                 125
   Ile Gly Pro Cys Lys Tyr Ile Pro Pro Cys Leu Asp Ser Glu Leu Thr
   130 135 140
   Glu Phe Pro Leu Arg Met Arg Asp Trp Leu Lys Asn Val Leu Val Thr
   145 150 155 160
   Leu Tyr Glu Arg Asp Glu Asp Asn Asn Leu Leu Thr Glu Lys Gln Lys
   165 170 175
   Leu Arg Val Lys Lys Ile His Glu Asn Glu Lys Arg Leu Glu Ala Gly
   180 185 190
   Asp His Pro Val Glu Leu Leu Ala Arg Asp Phe Glu Lys Asn Tyr Asn
   195 200 205
   Met Tyr Ile Phe Pro Val His Trp Gln Phe Gly Gln Leu Asp Gln His
   210 215 220
   Pro Ile Asp Gly Tyr Leu Ser His Thr Glu Leu Ala Pro Leu Arg Ala
   225 230 235 240
   Pro Leu Ile Pro Met Glu His Cys Thr Thr Arg Phe Phe Glu Thr Cys
   245 250 255
   Asp Leu Asp Asn Asp Lys Tyr Ile Ala Leu Asp Glu Trp Ala Gly Cys
   260 265 270
   Phe Gly Ile Lys Gln Lys Asp Ile Asp Lys Asp Leu Val Ile
   275 280 285
<210> 2
   <211> 3178
   <212> DNA
   <213> Homo sapiens
<400> 2
   gttgcctgtc tctaaacccc tccacattcc cgcggtcctt cagactgccc ggagagcgcg 60
   ctctgcctgc cgcctgcctg cctgccactg agggttccca gcaccatgag ggcctggatc 120
   ttctttctcc tttgcctggc cgggagggcc ttggcagccc ctcagcaaga agccctgcct 180
   gatgagacag aggtggtgga agaaactgtg gcagaggtga ctgaggtatc tgtgggagct 240
   aatcctgtcc aggtggaagt aggagaattt gatgatggtg cagaggaaac cgaagaggag 300
   gtggtggcgg aaaatccctg ccagaaccac cactgcaaac acggcaaggt gtgcgagctg 360
   gatgagaaca acacccccat gtgcgtgtgc caggacccca ccagctgccc agcccccatt 420
   ggcgagtttg agaaggtgtg cagcaatgac aacaagacct tcgactcttc ctgccacttc 480
   tttgccacaa agtgcaccct ggagggcacc aagaagggcc acaagctcca cctggactac 540
   atcgggcctt gcaaatacat ccccccttgc ctggactctg agctgaccga attccccctg 600
   cgcatgcggg actggctcaa gaacgtcctg gtcaccctgt atgagaggga tgaggacaac 660
   aaccttctga ctgagaagca gaagctgcgg gtgaagaaga tccatgagaa tgagaagcgc 720
   ctggaggcag gagaccaccc cgtggagctg ctggcccggg acttcgagaa gaactataac 780
   atgtacatct tccctgtaca ctggcagttc ggccagctgg accagcaccc cattgacggg 840
   tacctctccc acaccgagct ggctccactg cgtgctcccc tcatccccat ggagcattgc 900
   accacccgct ttttcgagac ctgtgacctg gacaatgaca agtacatcgc cctggatgag 960
   tgggccggct gcttcggcat caagcagaag gatatcgaca aggatcttgt gatctaaatc 1020
   cactccttcc acagtaccgg attctctctt taaccctccc cttcgtgttt cccccaatgt 1080
   ttaaaatgtt tggatggttt gttgttctgc ctggagacaa ggtgctaaca tagatttaag 1140
   tgaatacatt aacggtgcta aaaatgaaaa ttctaaccca agacatgaca ttcttagctg 1200
   taacttaact attaaggcct tttccacacg cattaatagt cccatttttc tcttgccatt 1260
   tgtagctttg cccattgtct tattggcaca tgggtggaca cggatctgct gggctctgcc 1320
   ttaaacacac attgcagctt caacttttct ctttagtgtt ctgtttgaaa ctaatactta 1380
   ccgagtcaga ctttgtgttc atttcatttc agggtcttgg ctgcctgtgg gcttccccag 1440
   gtggcctgga ggtgggcaaa gggaagtaac agacacacga tgttgtcaag gatggttttg 1500
   ggactagagg ctcagtggtg ggagagatcc ctgcagaacc caccaaccag aacgtggttt 1560
   gcctgaggct gtaactgaga gaaagattct ggggctgtgt tatgaaaata tagacattct 1620
   cacataagcc cagttcatca ccatttcctc ctttaccttt cagtgcagtt tcttttcaca 1680
   ttaggctgtt ggttcaaact tttgggagca cggactgtca gttctctggg aagtggtcag 1740
   cgcatcctgc agggcttctc ctcctctgtc ttttggagaa ccagggctct tctcaggggc 1800
   tctagggact gccaggctgt ttcagccagg aaggccaaaa tcaagagtga gatgtagaaa 1860
   gttgtaaaat agaaaaagtg gagttggtga atcggttgtt ctttcctcac atttggatga 1920
   ttgtcataag gtttttagca tgttcctcct tttcttcacc ctcccctttt ttcttctatt 1980
   aatcaagaga aacttcaaag ttaatgggat ggtcggatct cacaggctga gaactcgttc 2040
   acctccaagc atttcatgaa aaagctgctt cttattaatc atacaaactc tcaccatgat 2100
   gtgaagagtt tcacaaatcc ttcaaaataa aaagtaatga cttagaaact gccttcctgg 2160
   gtgatttgca tgtgtcttag tcttagtcac cttattatcc tgacacaaaa acacatgagc 2220
   atacatgtct acacatgact acacaaatgc aaacctttgc aaacacatta tgcttttgca 2280
   cacacacacc tgtacacaca caccggcatg tttatacaca gggagtgtat ggttcctgta 2340
   agcactaagt tagctgtttt catttaatga cctgtggttt aacccttttg atcactacca 2400
   ccattatcag caccagactg agcagctata tccttttatt aatcatggtc attcattcat 2460
   tcattcattc acaaaatatt tatgatgtat ttactctgca ccaggtccca tgccaagcac 2520
   tggggacaca gttatggcaa agtagacaaa gcatttgttc atttggagct tagagtccag 2580
   gaggaataca ttagataatg acacaatcaa atataaattg caagatgtca caggtgtgat 2640
   gaagggagag taggagagac catgagtatg tgtaacagga ggacacagca ttattctagt 2700
   gctgtactgt tccgtacggc agccactacc cacatgtaac tttttaagat ttaaatttaa 2760
   attagttaac attcaaaacg cagctcccca atcacactag caacatttca agtgcttgag 2820
   agccatgcat gattagtggt taccctattg aataggtcag aagtagaatc ttttcatcat 2880
   cacagaaagt tctattggac agtgctcttc tagatcatca taagactaca gagcactttt 2940
   caaagctcat gcatgttcat catgttagtg tcgtattttg agctggggtt ttgagactcc 3000
   ccttagagat agagaaacag acccaagaaa tgtgctcaat tgcaatgggc cacataccta 3060
   gatctccaga tgtcatttcc cctctcttat tttaagttat gttaagatta ctaaaacaat 3120
   aaaagctcct aaaaaatcaa aaaaaaaaaa aaaaaaaaaa aaaaaaaaaa aaaaaaaa 3178
<210> 3
   <211> 285
   <212> PRT
   <213> Homo sapiens
<400> 3
   Ala Pro Gln Glu Ala Leu Pro Asp Glu Thr Glu Val Val Glu Glu Thr
   1               5                   10                  15
   Val Ala Glu Val Thr Glu Val Ser Val Gly Ala Asn Pro Val Gln Val
               20                  25                  30
   Glu Val Gly Glu Phe Asp Asp Gly Ala Glu Glu Thr Glu Glu Glu Val
               35                  40                  45
   Val Ala Glu Asn Pro Cys Gln Asn His His Cys Lys His Gly Lys Val
       50                  55                  60
   Cys Glu Leu Asp Glu Asn Asn Thr Pro Met Cys Val Cys Gln Asp Pro
   65                  70                  75                  80
   Thr Ser Cys Pro Ala Pro Ile Gly Glu Phe Glu Lys Val Cys Ser Asn
                   85                  90                  95
   Asp Asn Lys Thr Phe Asp Ser Ser Cys His Phe Phe Ala Thr Lys Cys
               100                 105                 110
   Thr Leu Glu Gly Thr Lys Lys Gly His Lys Leu His Leu Asp Tyr Ile
           115                 120                 125
   Gly Pro Cys Lys Tyr Ile Pro Pro Cys Leu Asp Ser Glu Leu Thr Glu
       130                 135                 140
   Phe Pro Leu Arg Met Arg Asp Trp Leu Lys Asn Val Leu Val Thr Leu
   145                 150                 155                 160
   Tyr Glu Arg Asp Glu Asp Asn Asn Leu Leu Thr Glu Lys Gln Lys Leu
                   165                 170                 175
   Arg Val Lys Lys Ile His Glu Asn Glu Lys Arg Leu Glu Ala Gly Asp
               180                 185                 190
   His Pro Val Glu Leu Leu Ala Arg Asp Phe Glu Lys Asn Tyr Asn Met
           195                 200                 205
   Tyr Ile Phe Pro Val His Trp Gln Phe Gly Gln Leu Asp Gln His Pro
       210                 215                 220
   Ile Asp Gly Tyr Leu Ser His Thr Glu Leu Ala Pro Leu Arg Ala Pro
   225                 230                 235                 240
   Leu Ile Pro Met Glu His Cys Thr Thr Arg Phe Phe Glu Thr Cys Asp
                   245                 250                 255
   Leu Asp Asn Asp Lys Tyr Ile Ala Leu Asp Glu Trp Ala Gly Cys Phe
               260                 265                 270
   Gly Ile Lys Gln Lys Asp Ile Asp Lys Asp Leu Val Ile
   275 280 285
<210> 4
   <211> 2956
   <212> DNA
   <213> Homo sapiens
<400> 4
   ctccacattc ccgcggtcct tcagactgcc cggagagcgc gctctgcctg ccgcctgcct 60
   gcctgccact gagggttccc agcaccatga gggcctggat cttctttctc ctttgcctgg 120
   ccgggagggc cttggcagcc cctcaagaag ccctgcctga tgagacagag gtggtggaag 180
   aaactgtggc agaggtgact gaggtatctg tgggagctaa tcctgtccag gtggaagtag 240
   gagaatttga tgatggtgca gaggaaaccg aagaggaggt ggtggcggaa aatccctgcc 300
   agaaccacca ctgcaaacac ggcaaggtgt gcgagctgga tgagaacaac acccccatgt 360
   gcgtgtgcca ggaccccacc agctgcccag cccccattgg cgagtttgag aaggtgtgca 420
   gcaatgacaa caagaccttc gactcttcct gccacttctt tgccacaaag tgcaccctgg 480
   agggcaccaa gaagggccac aagctccacc tggactacat cgggccttgc aaatacatcc 540
   ccccttgcct ggactctgag ctgaccgaat tccccctgcg catgcgggac tggctcaaga 600
   acgtcctggt caccctgtat gagagggatg aggacaacaa ccttctgact gagaagcaga 660
   agctgcgggt gaagaagatc catgagaatg agaagcgcct ggaggcagga gaccaccccg 720
   tggagctgct ggcccgggac ttcgagaaga actataacat gtacatcttc cctgtacact 780
   ggcagttcgg ccagctggac cagcacccca ttgacgggta cctctcccac accgagctgg 840
   ctccactgcg tgctcccctc atccccatgg agcattgcac cacccgcttt ttcgagacct 900
   gtgacctgga caatgacaag tacatcgccc tggatgagtg ggccggctgc ttcggcatca 960
   agcagaagga tatcgacaag gatcttgtga tctaaatcca ctccttccac agtaccggat 1020
   tctctcttta accctcccct tcgtgtttcc cccaatgttt aaaatgtttg gatggtttgt 1080
   tgttctgcct ggagacaagg tgctaacata gatttaagtg aatacattaa cggtgctaaa 1140
   aatgaaaatt ctaacccaag acatgacatt cttagctgta acttaactat taaggccttt 1200
   tccacacgca ttaatagtcc catttttctc ttgccatttg tagctttgcc cattgtctta 1260
   ttggcacatg ggtggacacg gatctgctgg gctctgcctt aaacacacat tgcagcttca 1320
   acttttctct ttagtgttct gtttgaaact aatacttacc gagtcagact ttgtgttcat 1380
   ttcatttcag ggtcttggct gcctgtgggc ttccccaggt ggcctggagg tgggcaaagg 1440
   gaagtaacag acacacgatg ttgtcaagga tggttttggg actagaggct cagtggtggg 1500
   agagatccct gcagaaccca ccaaccagaa cgtggtttgc ctgaggctgt aactgagaga 1560
   aagattctgg ggctgtgtta tgaaaatata gacattctca cataagccca gttcatcacc 1620
   atttcctcct ttacctttca gtgcagtttc ttttcacatt aggctgttgg ttcaaacttt 1680
   tgggagcacg gactgtcagt tctctgggaa gtggtcagcg catcctgcag ggcttctcct 1740
   cctctgtctt ttggagaacc agggctcttc tcaggggctc tagggactgc caggctgttt 1800
   cagccaggaa ggccaaaatc aagagtgaga tgtagaaagt tgtaaaatag aaaaagtgga 1860
   gttggtgaat cggttgttct ttcctcacat ttggatgatt gtcataaggt ttttagcatg 1920
   ttcctccttt tcttcaccct cccctttttt cttctattaa tcaagagaaa cttcaaagtt 1980
   aatgggatgg tcggatctca caggctgaga actcgttcac ctccaagcat ttcatgaaaa 2040
   agctgcttct tattaatcat acaaactctc accatgatgt gaagagtttc acaaatcctt 2100
   caaaataaaa agtaatgact tagaaactgc cttcctgggt gatttgcatg tgtcttagtc 2160
   ttagtcacct tattatcctg acacaaaaac acatgagcat acatgtctac acatgactac 2220
   acaaatgcaa acctttgcaa acacattatg cttttgcaca cacacacctg tacacacaca 2280
   ccggcatgtt tatacacagg gagtgtatgg ttcctgtaag cactaagtta gctgttttca 2340
   tttaatgacc tgtggtttaa cccttttgat cactaccacc attatcagca ccagactgag 2400
   cagctatatc cttttattaa tcatggtcat tcattcattc attcattcac aaaatattta 2460
   tgatgtattt actctgcacc aggtcccatg ccaagcactg gggacacagt tatggcaaag 2520
   tagacaaagc atttgttcat ttggagctta gagtccagga ggaatacatt agataatgac 2580
   acaatcaaat ataaattgca agatgtcaca ggtgtgatga agggagagta ggagagacca 2640
   tgagtatgtg taacaggagg acacagcatt attctagtgc tgtactgttc cgtacggcag 2700
   ccactaccca catgtaactt tttaagattt aaatttaaat tagttaacat tcaaaacgca 2760
   gctccccaat cacactagca acatttcaag tgcttgagag ccatgcatga ttagtggtta 2820
   ccctattgaa taggtcagaa gtagaatctt ttcatcatca cagaaagttc tattggacag 2880
   tgctcttcta gatcatcata agactacaga gcacttttca aagctcatgc atgttcatca 2940
   tgttagtgtc gtattt 2956

## Description

### BACKGROUND OF THE INVENTION

The anticancer agent paclitaxel, marketed under the trademark Taxol^{®} by Bristol Myers Squibb, is currently approved for the treatment of several cancers including ovarian, lung, and breast cancer. A major limitation to the use of paclitaxel is its poor solubility. Consequently, the Taxol^{®} formulation contains Cremophor^{®} EL as the solubilizing vehicle, but the presence of Cremophor^{®} in this formulation has been linked to severe hypersensitivity reactions in animals (Lorenz et al., Agents Actions 7, 63-67, 1987), and humans (Weiss et al., J. Clin. Oncol. 8, 1263-1268, 1990). Accordingly, patients receiving Taxol^{®} require premedication with corticosteroids (dexamethasone) and antihistamines to reduce the hypersensitivity and anaphylaxis that occurs due to the presence of Cremophor^{®}.

In contrast, Abraxane^{®}, also known as ABI-007, is a Cremophor^{®}-free, albumin-nanoparticle formulation of paclitaxel, marketed by Abraxis Oncology. The use of an albumin nanoparticle as a vehicle results in the formation of a colloid when reconstituted with saline. Based on clinical studies, it has been shown that the use of Abraxane^{®} is characterized by reduced hypersensitivity reactions as compared with Taxol^{®}. Accordingly, premedication is not required for patients receiving Abraxane^{®}.

Another advantage of the albumin-nanoparticle formulation is that by excluding toxic emulsifiers it is possible to administer higher doses of paclitaxel at more frequent intervals than is currently possible with Taxol^{®}. The potential exists that enhanced efficacy could be seen in solid tumors as a consequence of (i) higher tolerable doses (300 mg/m²), (ii) longer half-life, (iii) prolonged local tumor availability and/or (iv) sustained *in vivo* release. Abraxane^{®} reduces hypersensitivity reactions while maintaining or improving the chemotherapeutic effect of the drug.

Secreted Protein, Acidic, Rich in Cysteines (SPARC), also known as osteonectin, is a matricellular glycoprotein which facilitates Abraxane^{®} activity. SPARC has affinity for a wide variety of ligands including cations (e.g., Ca²⁺, Cu²⁺, Fe⁺), growth factors (e.g., platelet derived growth factor (PDGF), and vascular endothelial growth factor (VEGF)), extracellular matrix (ECM) proteins (e.g., collagen I-V and collagen IX, vitronectin, and thrombospondin-1), endothelial cells, platelets, hydroxyapaptite and, most importantly for the current application, albumin. SPARC expression is developmentally regulated, and is predominantly expressed in tissues undergoing remodeling during normal development or in response to injury (see, e.g., Lane et al., FASEB J., 8, 163-173 (1994)). High levels of SPARC protein are expressed in developing bones and teeth. SPARC is also upregulated in several aggressive cancers, but is absent from the vast majority of normal tissues (Porter et al., J. Histochem. Cytochem., 43, 791(1995) and see below). Indeed, SPARC expression is induced among a variety of tumors (e.g., bladder, liver, ovary, kidney, gut, and breast).

The closest prior art documents are WO 2004/064785, WO 2006/058425 and WO 2008/000079. WO 2004/064785 relates generally to compositions and methods for sensitizing cancer therapy. WO 2006/058425 relates generally to oligonucleotide or peptide nucleic acids, arrays, cells, methods and kits for determining a cancer's resistance or sensitivity to a therapeutic regimen. WO 2008/000079 relates generally to compositions and methods of use thereof for cancer therapy sensitization. Tai, I.T. et al. (J. Clin. Invest. (2005):115(6); p. 1492-1502) generally describe the resistance reversing effect of the SPARC polypeptide on therapy resistant tumors. Desai N. et al. (Breast Cancer Research and Treatment, Vol. 88, no. Suppl. 1, 2004, pages S26-S27) disclose generally that that some aggressive tumors such as breast tumors overexpress SPARC and compare albumin-bound paclitaxel (ABX) in nanoparticle form with Taxol in these settings. Gradishar W.J. (Expert Opinion on Pharmacotherapy (2006):7(8); p. 1041-53) discloses a solvent-free formulation of paclitaxel for the treatment of metastatic breast cancer.

There remains a need for better methods of treating human and other mammalian tumors, as well as other proliferative, hyperplastic, remodeling, and inflammatory disorders.

### BRIEF SUMMARY OF THE INVENTION

The invention provides compositions for treating a mammalian tumor comprising a SPARC polypeptide, an angiogenesis inhibitor and albumin bound paclitaxel , optionally with a suitable carrier. Further, the disclosure provides methods for treating a mammalian tumor comprising a therapeutically effective amount of this composition

In preferred embodiments the albumin bound paclitaxel is an albumin bound paclitaxel with 50% of the paclitaxel in nanoparticle form. In preferred embodiments the angiogenesis inhibitor is avastin, sutent or sorafenib.

### BRIEF DESCRIPTION OF THE DRAWINGS

FIG. 1 depicts the competitive SPARC binding to albumin.

FIG. 2 illustrates albumin and SPARC staining in MX 1 tumor xenografts.

FIG. 3 depicts transcytosis of paclitaxel across endothelial cell monolayers.

FIG. 4 compares the growth of tumor xenografts comparing cancer cells expressing or not expressing wild type SPARC (SEQ ID NO: 1), in the presence or absence of Abraxane^{®}.

FIG. 5A compares the growth of tumor xenografts in the presence or absence of exogenous wild type SPARC (SEQ ID NO: 1) and in the presence or absence of 5-Flourouracil.

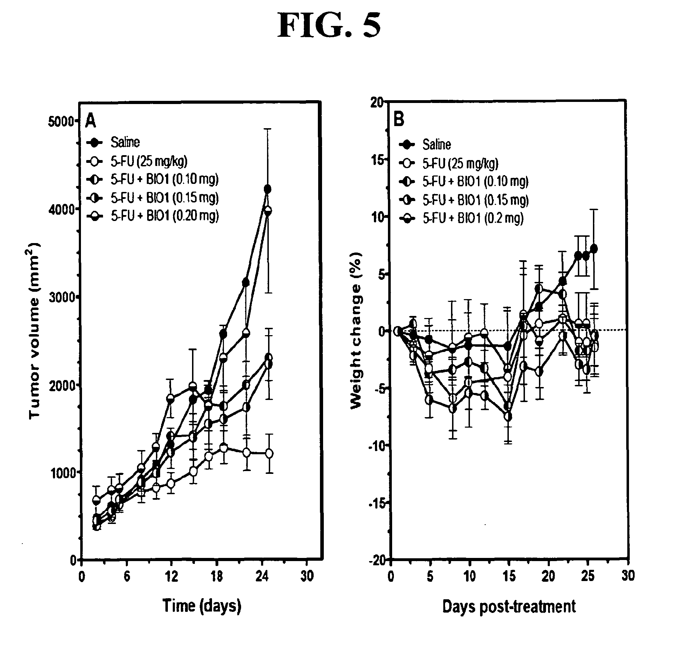

FIG. 5A compares the weight change of mice, in the presence or absence of exogenous wild type SPARC (SEQ ID NO: 1) and in the presence or absence of 5-Flourouracil.

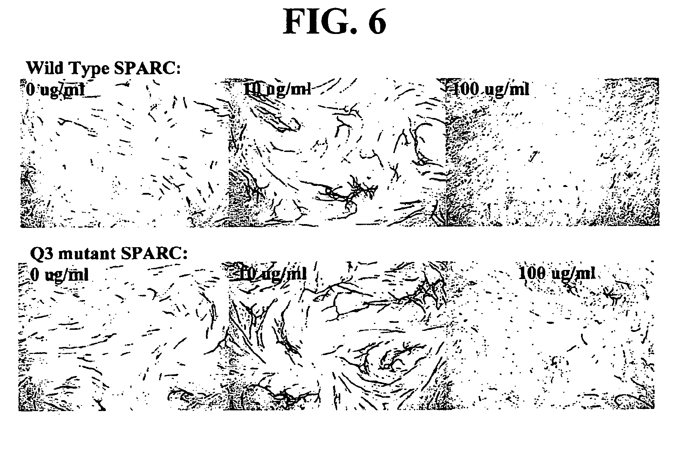

FIG. 6 depicts SPARC's concentration dependent effect on neoangiogensis.

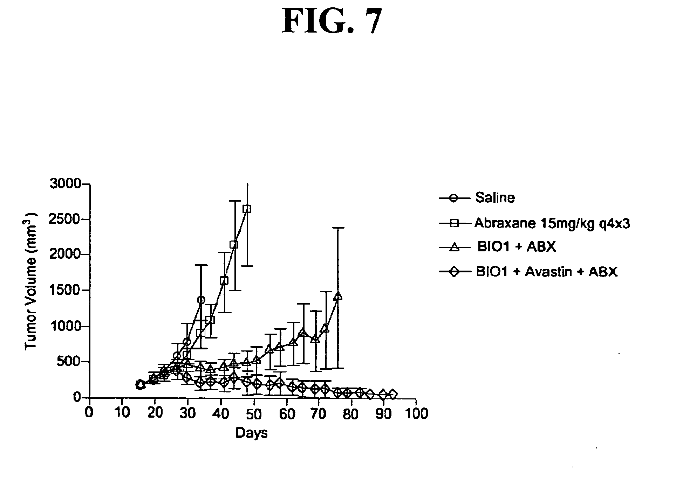

FIG. 7 compares the growth of breast cancer xenografts, in the presence or absence of exogenous wild type SPARC (SEQ ID NO: 1) in the presence or absence of the angiogenesis inhibitor avastin and in the presence or absence of Abraxane^{®}.

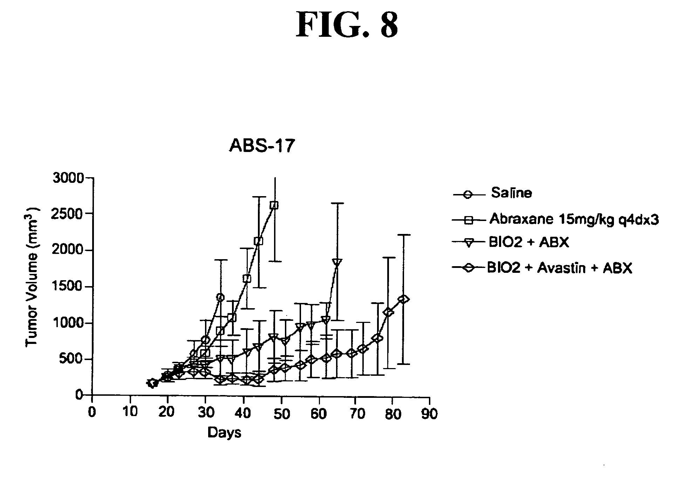

FIG. 8 compares the growth of breast cancer xenografts, in the presence or absence of exogenous Q3 mutant SPARC (SEQ ID NO: 3) in the presence or absence the angiogenesis inhibitor avastin and in the presence or absence of Abraxane^{®}.

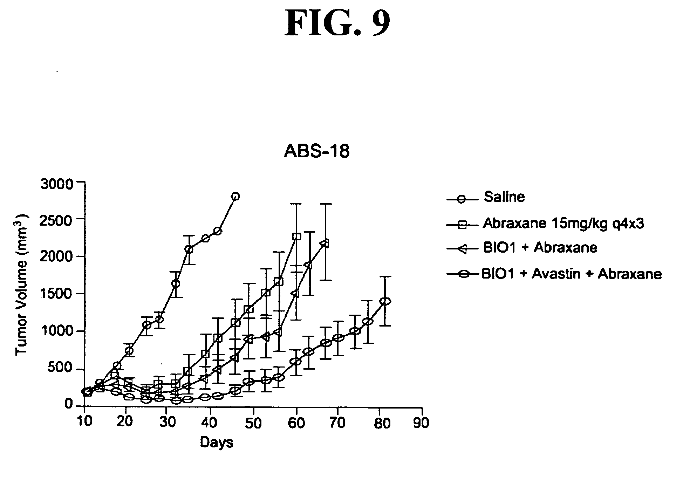

FIG. 9 compares the growth of colon cancer xenografts, in the presence or absence of exogenous wild type SPARC (SEQ ID NO: 1) in the presence or absence of the angiogenesis inhibitor avastin and in the presence or absence of Abraxane^{®}.

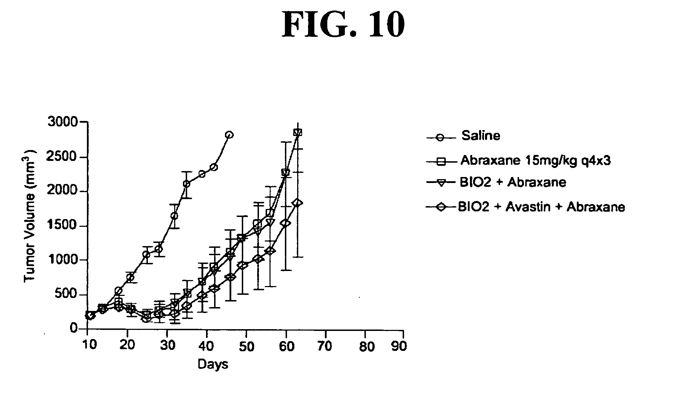

FIG. 10 compares the growth of colon cancer xenografts, in the presence or absence of exogenous Q3 mutant SPARC (SEQ ID NO: 3) in the presence or absence of the angiogenesis inhibitor avastin and in the presence or absence of Abraxane^{®}.

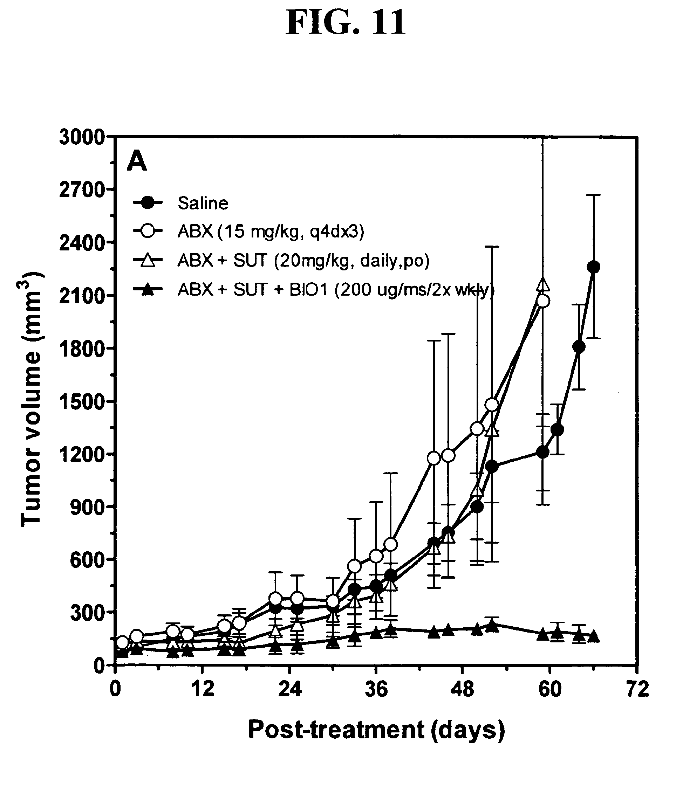

FIG. 11 compares the growth of colon cancer xenografts, in the presence or absence of exogenous Q3 mutant SPARC (SEQ ID NO: 3) in the presence or absence of Stutan as anangiogenesis inhibitor and in the presence or absence of Abraxane^{®}.

### DETAILED DESCRIPTION OF THE INVENTION

I. Therapeutic Compositions and Methods

While not wanting to bound by any particular theory, the effectiveness of the aspects of the invention employing angiogenesis inhibitor may be related to the inventors' unexpected and surprising findings that exogenous SPARC's angiogenic activity varies in a concentration-dependent manner from pro-angiogenic to anti-angiogenic and that, at low concentrations, exogenous SPARC's pro-angiogenic activity can mask its other anti-tumor activities. Thus, an angiogenesis inhibitor may unmask SPARC anti-tumor activities based on other mechanisms.

The human SPARC gene encodes a 303 amino acid SPARC protein, while mature SPARC is a 285 amino acid glycoprotein. After cleavage of the signal sequence a 32-kD secreted form is produced which migrates at 43 kD on SDS-PAGE because of glycosylation. The amino acid sequence of the complete SPARC mature protein is disclosed in SEQ ID NO: 1 and the nucleic acid sequence of an RNA encoding such a SPARC protein is disclosed in SEQ ID NO: 2 (presented as a cDNA sequence, i.e., with the RNA uridines ("U") as thymines ("T")). An alternative form of SPARC, the Q3 mutant (SEQ ID NO: 3), has been discovered which has a mutation corresponding to a deletion of the third glutamine in the mature form of the human SPARC protein. SEQ ID NO: 4 is a nucleic acid encoding the Q3 mutant polypeptide. See, U.S. Patent No. 7,332,568.

As used herein the terms "polypeptide" and "protein" are used interchangeably. The invention provides for the use, production, detection and quantification of a SPARC polypeptide or protein such as, e.g., a polypeptide or protein comprising an amino acid sequence of SEQ ID NO: 1 or 3. The invention also provides for the use, production, detection and quantification of SPARC polypeptide, wherein the polypeptide comprises an amino acid sequence of at least about 10 sequential amino acids from the sequence of SEQ ID NO: 1 or 3, preferably at least about 15 sequential amino acids from the sequence of SEQ ID NO: 1 or 3, more preferably at least about 20 sequential amino acids from the sequence of SEQ ID NO: 1 or 3, and most preferably at least about 100 sequential amino acids from the sequence of SEQ ID NO: 1 or 3. Further, the invention provides for the detection of a SPARC polypeptide comprising a polypeptide wherein the sequence is at least about 80% identical to the corresponding sequence of SEQ ID NO: 1 or 3, preferably at least about 90% identical to the corresponding sequence of SEQ ID NO: 1 or 3, even more preferably at least about 95% identical to the corresponding sequence of SEQ ID NO: 1 or 3, and even more preferably at least about 99% identical to the corresponding sequence of SEQ ID NO: 1 or 3.

By, for example "corresponding sequence of SEQ ID NO: 1" it is meant, the sequence which aligns with the sequence of SEQ ID NO: 1 wherein the region of alignment is at least about 10 amino acids long, preferably is at least about 15 amino acids long, more preferably is at least about 20 amino acids long, more preferably is at least about 30 amino acids long, more preferably is at least about 40 amino acids long, more preferably is at least about 50 amino acids long, and even more preferably is at least about 100 amino acids long. The "corresponding sequence of SEQ ID NO: 2" is defined as the sequence which aligns with the sequence of SEQ ID NO: 3 wherein the region of alignment is at least about 10 amino acids long, preferably is at least about 15 amino acids long, more preferably is at least about 20 amino acids long, more preferably is at least about 30 amino acids long, more preferably is at least about 40 amino acids long, more preferably is at least about 50 amino acids long, and even more preferably is at least about 100 amino acids long.. Various methods of sequence alignment are known in the biotechnology arts (see, e.g., Rosenberg, BMC Bioinformatics 6:278 (2005); Altschul et al., FEBS J. 272(20): 5101-5109 (2005)).

Suitable SPARC polypeptides for use in accordance with the invention may also be comprised of a polypeptide as described herein with significant sequence identity with SEQ ID NO: 1 or 3 and an additional about 5, preferably an additional about 10, more preferably an additional about 25, even more preferably an additional about 50, or most preferably an additional about 100 amino acids at its amino and/or carboxyl termini.

The invention provides for the use, cloning, expression, detection and quantification of a SPARC RNA such, e.g., an RNA comprising the nucleic acid sequence corresponding to the cDNA of SEQ ID NO: 2 or 4. The invention also provides for the detection of SPARC RNA, wherein the RNA comprises the nucleic sequence of at least about 15 sequential nucleotides from the sequence of SEQ ID NO: 2 or 4, preferably at least about 20 sequential nucleotides from the sequence of SEQ ID NO: 2, and more preferably at least about 30 sequential nucleotides from the sequence of SEQ ID NO: 2 or 4. Further, the invention provides for the detection of a SPARC RNA comprising a nucleic acid wherein the sequence is at least about 80% identical to the corresponding sequence of SEQ ID NO: 2 or 4 , preferably at least about 90% identical to the corresponding sequence of SEQ ID NO: 2 or 4, even more preferably at least about 95% identical to the corresponding sequence of SEQ ID NO: 2 or 4, and even more preferably at least about 99% identical to the corresponding sequence of SEQ ID NO: 2 or 4. For example, by "corresponding sequence of SEQ ID NO: 2" it is meant, the sequence which aligns with the sequence of SEQ ID NO: 2 wherein the region of alignment is at least about 15 nucleotides long, preferably is at least about 20 nucleotides long, more preferably is at least about 30 nucleotides long, more preferably is at least about 60 nucleotides long, more preferably is at least about 120 nucleotides long, more preferably is at least about 150 nucleotides long, even more preferably is at least about 200 nucleotides long. Various methods of sequence alignment are known in the biotechnology arts (see, e.g., Rosenberg, BMC Bioinformatics 6:278 (2005); Altschul et al., FEBS J. 272(20): 5101-5109 (2005)). By SPARC RNA it is meant any SPARC RNA, including but, not limited to, a SPARC mRNA, hnRNA, primary transcript or splice variant.

By "therapeutically effective amount" it is meant an amount of a composition that relieves (to some extent, as judged by a skilled medical practitioner) one or more symptoms of the disease or condition in a mammal. Additionally, by "therapeutically effective amount" of a composition is meant an amount that returns to normal, either partially or completely, physiological or biochemical parameters associated with or causative of a disease or condition. A clinician skilled in the art can determine the therapeutically effective amount of a composition in order to treat or prevent a particular disease condition, or disorder when it is administered, such as intravenously, subcutaneously, intraperitoneally, orally, or through inhalation. The precise amount of the composition required to be therapeutically effective will depend upon numerous factors, e.g., such as the specific activity of the active agent, the delivery device employed, physical characteristics of the agent, purpose for the administration, in addition to many patient specific considerations. But, is within the skill of an ordinarily skilled clinician upon the appreciation of the disclosure set forth herein.

As used herein, "the response of a human or other mammalian tumor to a chemotherapeutic agent" refers the degree or amount that the patient improves clinically or that the tumor decreases in size or aggressiveness because of a chemotherapeutic agent. The patient can be said to improve clinically based on objective criteria, such as, e.g., performance status, physical examination, imaging studies or laboratory test results. The patient also can be said to improve clinically based subjective criteria reported by the patient, such as, e.g., pain, distress, fatigue or mental outlook. Decreases in size tumor size can be based on the primary tumor or overall tumor burden measured by any suitable method known in the art, e.g., physical examination, imaging study or laboratory value. By "decrease in tumor size" it is meant a change of at least about 10%. Further, it is desirable that a change of at least about 20% be present, preferably a change of at least about 25%, more preferably a change of at least about 33%, more preferably a change of at least about 50%, more preferably a change of at least about 90%, more preferably a change of at least about 95%, and most preferably a change of at least about 99%. By decrease in the "tumor aggressiveness" it is meant, e.g., a reduction the histologic grade, % viable cells in the tumor, % proliferating cells in the tumor, the tumor's invasiveness, the tumor's ability to metastasize or other metric of tumor aggressiveness know in the art. By "decrease in the tumor's aggressiveness" it is meant a change of at least about 10% in a measurable parameter related to tumor aggressiveness which is commonly used by those of ordinary skill in the medical arts, for example, without limitation, stage, grade, tumor burden, extent of metsastatic spread, vascularity, DNA content, and proliferative fraction. Further, it is desirable that a change of at least about 20% be present, preferably a change of at least about 25%, more preferably a change of at least about 33%, more preferably a change of at least about 50%, more preferably a change of at least about 90%, more preferably a change of at least about 95%, and most preferably a change of at least about 99% in a measurable parameter related to tumor aggressiveness which is commonly used by those of ordinary skill in the medical arts. The invention also provides a method for predicting or determining the response of a human or other mammalian tumor or other proliferative disease to a chemotherapeutic agent or other anticancer agent. The method comprises (a) isolating a biological sample from the human or other mammalian, (b) detecting the expression of SPARC protein in the biological sample, and (c) quantifying the amount of SPARC protein in the biological sample. Once the amount of SPARC expressed by the tumor is determined, the effectiveness of the chemotherapeutic agent can be predicted or ascertained by, for example, correlating of the expression of SPARC to the dosage of therapeutic agent administered. The invention also provides for the use of antibody raised against SPARC as a therapeutic agent, or an imaging agent for diseases where SPARC plays a dominant role and is overexpressed relative to normal tissues.

The terms "treating," "treatment," "therapy," and "therapeutic treatment" as used herein refer to curative therapy, prophylactic therapy, or preventative therapy. An example of "preventative therapy" is the prevention or lessening the chance of a targeted disease (e. g., cancer or other proliferative disease) or related condition thereto. Those in need of treatment include those already with the disease or condition as well as those prone to have the disease or condition to be prevented. The terms "treating," "treatment," "therapy," and "therapeutic treatment" as used herein also describe the management and care of a mammal for the purpose of combating a disease, or related condition, and includes the administration of a composition to alleviate the symptoms, side effects, or other complications of the disease, condition. Therapeutic treatment for cancer includes, but is not limited to, surgery, chemotherapy, radiation therapy, gene therapy, and immunotherapy.

As used herein, the term "agent" or "drug" or "therapeutic agent" refers to a chemical compound, a mixture of chemical compounds, a biological macromolecule, or an extract made from biological materials such as bacteria, plants, fungi, or animal (particularly mammalian) cells or tissues that are suspected of having therapeutic properties. The agent or drug may be purified, substantially purified or partially purified. An "agent", according to the present invention, also includes a radiation therapy agent. As used herein, the term "chemotherapeutic agent" refers to an agent with activity against cancer, neoplastic, and/or proliferative diseases.

The invention provides compositions where any one or all of the SPARC polypeptide, angiogenesis inhibitor, and albumin-bound paclitaxel, are lyophilized for reconstitution at the bedside into liquid dosage forms. Dosage forms in accordance with the invention include where, the SPARC polypeptide, the angiogenesis inhibitor and the microtubule inhibitor are included in a single dosage form.

The compositions provide by the invention include, e.g., from about 0.5 ml to about 4 ml aqueous or organic liquids with a microtubule inhibitor concentration of from about 10 mg/ml to about 100 mg/ml, preferably from about 1 mg/ml to about 10 mg/ml, more preferably from about 0.1 mg/ml to about 1 mg/ml. Such compositions can have SPARC polypeptides concentrations of from about 10 µg/ml to about 400 mg/ml, preferably from about 100 µg/ml to about 100 mg/ml, and more preferably from about 1 mg/ml to about 10 mg/ml. The angiogenesis inhibitor may be present at any suitable and therapeutically effective concentration, e.g., Avastin at a concentration of from about 10 mg/ml to about 50 mg/ml.

In preferred embodiments, the invention provides compositions and methods for treating a mammalian tumor comprising a therapeutically effective amount of a SPARC polypeptide, a therapeutically effective amount of a microtubule inhibitor, and, an angiogenesis inhibitor, SPARC polypeptide is a polypeptide comprising the amino acid sequence of SEQ ID NOS. 1, 3 or a combination thereof. In particularly preferred embodiments the SPARC polypeptide is a polypeptide comprising the amino acid sequence of SEQ ID NO: 1.

The SPARC polypeptide is administered at a dose of from about 40 µg to about 40 mg per dose with a dosing cycle of at least about 1 week. In other words, the SPARC dose is from about 40 µg/kg to about 40 mg/kg, preferably from about 0.1 mg/kg to about 100 mg/kg, more preferably from about 1 mg/kg to about 20 mg/kg.

Suitable angiogenesis inhibitors for use in accordance with the invention include, e.g., an inhibitor of mTOR, Aurora kinase, an inhibitor of VEGFR kinase, an inhibitor of PDGFR kinase, sorafenib, Sutent, Axitinib, Avastin, marimastat, bevacizumab, carboxyamidotriazole, TNP-470, CM101, IFN-α, IL-12, platelet factor-4, suramin, SU5416, thrombospondin, VEGFR antagonists, angiostatic steroids, cartilage-derived angiogenesis inhibitory factor, matrix metalloproteinase inhibitors, angiostatin, endostatin, 2-methoxyestradiol, tecogalan, thrombospondin, prolactin, αavβ3 inhibitors, tecogalan, BAY 12-9566, AG3340, CGS27023A, COL-3, vitaxin, ZD0101, TNP-40, thalidomide, squalamine, IM862, PTK787, fumagillin, analogues of fumagillin, BB-94, BB-2516, linomid, antibodies to vascular growth factors, antibodies to vascular growth factor receptors or combinations thereof.

Any suitable dose of angiogenesis inhibitor may be used, e.g., Avastin administered at a dose of from about 5 mg/kg to about 15 mg/kg with a dosing cycle of at least 1 week.

Hydrophobic chemotherapeutic agents have an HLB (HLB is hydrophilic/lipophilic balance number) of 1.0 or less, preferably 2.0 or less, most preferably 5.0 or less, and include, e.g. the agents epothilone, docetaxel, paclitaxel. Microtubule inhibitor such as taxanes include epothilone, docetaxel, paclitaxel, and combinations thereof. "Combinations thereof" refers to both the administration of dosage forms including more than one drug, for example, docetaxel and paclitaxel, as well as the sequential but, temporally distinct, administration of epothilone, docetaxel and paclitaxel (e.g., the use of docetaxel in one cycle and paclitaxel in the next). Particularly preferred chemotherapeutic agents comprise particles of protein-bound drug, including but not limited to, wherein the protein making up the protein-bound drug particles comprises albumin including wherein more than 50% of the chemotherapeutic agent is in nanoparticle form. Most preferably the chemotherapeutic agent comprises particles of albumin-bound paclitaxel, such as, e.g., Abraxane®.

Suitable nanoparticle formulations are not limited to those that comprise at least about 50% of the active agent in nanoparticle form. Other suitable nanoparticle formulations comprise at least about 60%, preferably at least about 70%, more preferably at least about 80%, or even more preferably at least about 90% of the active agent in nanoparticle form. Moreover, such nanoparticle formulations can most preferably comprise at least about 95% to at least about 98% of the active agent in nanoparticle form.

Methods of treating a mammalian tumor in accordance with this preferred aspect of the invention include, without limitation, wherein the paclitaxel dose is from about 30 mg/m² to about 1000 mg/m² with a dosing cycle of once about every week; preferably with a paclitaxel dose is from about 1 mg/m² to about 30 mg/m² with a dosing cycle of once about every week; more preferably with a paclitaxel dose is from about 0.3 mg/m² to about 1 mg/m² with a dosing cycle of once about every week most preferably with a paclitaxel dose is from about 0.01 mg/m² to about 0.3 mg/m² with a dosing cycle of once about every week. Suitable paclitaxel dosing cycles also include once every about 2 weeks or once every about 2 weeks.

Methods of treating a mammalian tumor in accordance with the invention include, without limitation, wherein the SPARC polypeptide is administered before, with or after the microtubule inhibitor. Similarly, the angiogenesis inhibitor can administered before, with or after the microtubule inhibitor such as taxane. By before or after, it is meant a time difference of at least about 2 weeks, preferably at least about 1 week, more preferably at least about 3 days, more preferably at least about 1 day, more preferably at least about 12 hours, more preferably at least about 8 hours, even more preferably at least about 6 hours, most preferably at least about 1 hour. By "substantially simultaneously" it is meant that the two events occur within about 3 days, preferably within about 1 day, more preferably within about 12 hours, more preferably within about 8 hours, even more preferably within about 6 hours, most preferably within about 1 hour.

Regimens in accordance with the invention include, about once weekly dose wherein:

(a) the SPARC polypeptide is at a dose of about 40 µg/kg to about 40 mg/kg per dose, the angiogenesis inhibitor is Avastin at a dose of from about 15 µg/kg to about 15 mg/kg, and the microtubule inhibitor is albumin-bound paclitaxel at a dose of from about 30 mg/m² to about 1000 mg/m²

(b) wherein the SPARC polypeptide is at a dose of about 40 µg/kg to about 40 mg/kg per dose, the angiogenesis inhibitor is Avastin at a dose of from about 15 µg/kg to about 15 mg/kg, and the microtubule inhibitor is albumin-bound paclitaxel at a dose of from about 1 mg/m² to about 30 mg/m² with a dosing cycle of at least 1 week..

(c) wherein the SPARC polypeptide is at a dose of about 40 µg/kg to about 40 mg/kg per dose, the angiogenesis inhibitor is Avastin at a dose of from about 15 µg/kg to about 15 mg/kg, and the microtubule inhibitor is albumin-bound paclitaxel at a dose of from about 0.3 mg/m² to about 1 mg/m² with a dosing cycle of at least 1 week..

(d) wherein the SPARC polypeptide is at a dose of about 40 µg/kg to about 40 mg/kg per dose, the angiogenesis inhibitor is Avastin at a dose of from about 15 µg/kg to about 15 mg/kg, and the microtubule inhibitor is albumin-bound paclitaxel at a dose of from about 0.1 mg/m² to about 0.3 mg/m² with a dosing cycle of at least 1 week..

The SPARC polypeptide in regimens (a)-(d) may have a sequence comprising of SEQ ID NO: 1, 2, or any other suitable SPARC and combinations thereof. These combinations can produce an about 50% reduction in the tumor growth rate compared to microtubule inhibitor such as taxane alone.

Additional therapies may be used with the SPARC polypeptide, angiogenesis inhibitor, and microtubule inhibitor, include suitable chemotherapeutic agents, e.g, tyrosine kinase inhibitors (genistein), biologically active agents (TNF, of tTF), radionuclides (¹³¹I, ⁹⁰Y, ¹¹¹In, ²¹¹At, ³²P and other known therapeutic radionuclides), adriamycin, ansamycin antibiotics, asparaginase, bleomycin, busulphan, cisplatin, carboplatin, carmustine, capecitabine, chlorambucil, cytarabine, cyclophosphamide, camptothecin, dacarbazine, dactinomycin, daunorubicin, dexrazoxane, docetaxel, doxorubicin, etoposide, epothilones, floxuridine, fludarabine, fluorouracil, gemcitabine, hydroxyurea, idarubicin, ifosfamide, irinotecan, lomustine, mechlorethamine, mercaptopurine, meplhalan, methotrexate, rapamycin (sirolimus) and derivatives, mitomycin, mitotane, mitoxantrone, nitrosurea, pamidronate, pentostatin, plicamycin, procarbazine, rituximab, streptozocin, teniposide, thioguanine, thiotepa, taxanes, vinblastine, vincristine, vinorelbine, combretastatins, discodermolides, and transplatinum. Accordingly, suitable chemotherapeutic agents for use in accordance with invention include, without limitation, antimetabolites (e.g., asparaginase), antimitotics (e.g., vinca alkaloid), DNA damaging agents (e.g., cisplatin), proapoptotics (agents which induce programmed-cell-death or apoptosis) (e.g, epipodophylotoxins), differentiation inducing agents (e.g., retinoids), antibiotics (e.g., bleomycin), and hormones (e.g., tamoxifen, diethylstibestrol). Further, suitable chemotherapeutic agents for use in accordance with the invention include antiangiogenesis agents (angiogenesis inhibitors) such as, e.g., INF-alpha, fumagillin, angiostatin, endostatin, thalidomide, and the like. "Other anticancer agents" also include, without limitation, biologically active polypeptides, antibodies, lectins, and toxins. Suitable antibodies for use in accordance with the invention include, without limitation, conjµgated (coupled) or unconjµgated (uncoupled) antibodies, monoclonal or polyclonal antibodies, humanized or unhumanized antibodies, as well as Fab', Fab, or Fab2 fragments, single chain antibodies and the like.

Diseases for which the present invention is useful include abnormal conditions of proliferation, tissue remodeling, hyperplasia, exaggerated wound healing in any bodily tissue including soft tissue, connective tissue, bone, solid organs, blood vessel and the like. Examples of diseases treatable or diagnosed by invention compositions include cancer, diabetic or other retinopathy, inflammation, arthritis, restenosis in blood vessels or artificial blood vessel grafts or intravascular devices and the like.

The invention also provides for a means of transporting the therapeutic composition across the endothelial barrier from the blood vessel into the tumor interstitium. The main hurdle in antibody therapy and chemotherapy is the translocation across the endothelial barrier into tumor interstitium. Albumin utilizes the albumin receptor transport mechanism to cross the endothelial barrier. This transport mechanism could be the same as those reported by the literature (gp60 and albondin) or by other undiscovered mechanisms. It has been previously reported that the therapeutic agent piggy backed onto albumin exhibited enhanced tumoral uptake (Desai, N. et al. Increased endothelial transcytosis of nanoparticle albumin-bound paclitaxel (ABI-007) by endothelial gp60 receptors: a pathway inhibited by Taxol®, 27th Annual San Antonio Breast Cancer Symposium (SABCS) (2004), abstract #1071). Further, enhanced translocation across the endothelial barrier can be achieved using the physiological albumin transport mechanism (Schnitzer, J.E.; Oh, P. J. Biol. Chem. 269, 6072-6082 (1994).

For small molecules, such as e.g., <1,000-5,000 Daltons, modifications can be made so that the drug affinity for albumin is increased. For formulations of small molecules, a solvent which prevents the binding of the drug to albumin may be removed. Alternatively, the small molecule may be linked to albumin, antibody against albumin, fragments thereof or ligands for an albumin-receptor such as described below.

For biologic molecules such as proteins, antibodies and fragments thereof, it is possible to engineer the biologics with an albumin binding peptide such that the biologics will exhibit an affinity for albumin. The peptide can either be an albumin binding sequence, an antibody or antibody fragment against albumin, antibody or antibody fragment against albumin carriers (such as gp60/albondin/scavenger receptor/or TGF-beta receptor), or antibody to any of the proteins found in the caveolae, the transporter of albumin.

SPARC may be synthesized and purified using known technologies. Cells expressing exogenous SPARC can be generated by placing the SPARC structural gene/cDNA under the control of strong promoter/translation start and the vector transfected into mammalian cells to drive the expression of SPARC in these cells. Alternatively, SPARC can be expressed using bacculovirus or other viruses such as adenovirus. SPARC expressed by these cells can be purified by traditional purifications methods such as ionic exchange, size exclusion, or C18 chromatography. The purified SPARC can be formulated in saline with preservatives and administered intravenously, by aerosol, by subcutaneous injection, or other methods.

The invention further provides for a recombinant vector comprising the nucleic acid sequence encoding, wherein, e.g., the vector further comprising a promoter controlling the expression of the SPARC polypeptide encoding nucleic acid sequences. In addition, the invention provides for a cell comprising the nucleic acid molecule of claim 3, wherein the cell is a prokaryotic cell or a eukaryotic cell. Methods of tissue culture are well known to the skilled artisan (see, e.g., Sambrook & Russell, Molecular Cloning: A Laboratory Manual, Cold Spring Harbor Laboratory Press, New York (2001), pp. 16.1-16.54). Accordingly, the invention further provides for method of making the polypeptide of claim 1 comprising: (a) transforming cells with a nucleic acid encoding the polypeptide of claim 1; (b) inducing the expression of the polypeptide by the transformed cells; and (c) purifying the polypeptide.

A SPARC polypeptide may be expressed and purified from a recombinant host cell. Recombinant host cells may be prokaryotic or eukaryotic, including but not limited to bacteria such as E. coli, fungal cells such as yeast, insect cells including but, not limited to, drosophila and silkworm derived cell lines, and mammalian cells and cell lines. When expressing a SPARC polypeptide in a cell, e.g., a human cell, whether, in vitro or in vivo, the codons selected for such the polynucleotide encoding the SPARC may be optimized for a given cell type (i.e., species). Many techniques for codon optimization are known in the art (see, e.g., Jayaraj et al, Nucleic Acids Res. 33(9):3011-6 (2005); Fµglsang et al., Protein Expr. Purif. 31(2):247-9 (2003); Wu et al., "The Synthetic Gene Designer: a Flexible Web Platform to Explore Sequence Space of Synthetic Genes for Heterologous Expression," csbw, 2005 IEEE Computational Systems Bioinformatics Conference--Workshops (CSBW'05), pp. 258-259 (2005)).

The invention further provides nucleic acid constructs comprising control elements and a SPARC polypeptide nucleic acid molecule described herein operatively linked to the control elements (e.g., a suitable promoter) for expression of a SPARC polypeptide or a polypeptide herein described with conservative amino acid changes in a SPARC polypeptide. Protein expression is dependent on the level of RNA transcription, which is in turn regulated by DNA signals. Similarly, translation of mRNA requires, at the very least, an ATG initiation codon, which is usually located within 10 to 100 nucleotides of the 5' end of the message. Sequences flanking the ATG initiator codon have been shown to influence its recognition by eukaryotic ribosomes, with conformity to a perfect Kozak consensus sequence resulting in optimal translation (see, e.g., Kozak, J. Molec. Biol. 196: 947-950 (1987)). Also, successful expression of an exogenous nucleic acid in a cell can require post-translational modification of a resultant protein. Accordingly, the invention provides plasmids encoding SPARC polypeptides wherein the vector is, e.g., pCDNA3.1 or a derivative thereof, and including but, not limited to, the pVT1000Q3 plasmid disclosed herein.

The nucleic acid molecules described herein preferably comprise a coding region operatively linked to a suitable promoter, which promoter is preferably functional in eukaryotic cells. Viral promoters, such as, without limitation, the RSV promoter and the adenovirus major late promoter can be used in the invention. Suitable non-viral promoters include, but are not limited to, the phosphoglycerokinase (PGK) promoter and the elongation factor 1.alpha. promoter. Non-viral promoters are desirably human promoters. Additional suitable genetic elements, many of which are known in the art, also can be ligated to, attached to, or inserted into the inventive nucleic acid and constructs to provide additional functions, level of expression, or pattern of expression. The native promoters for expression of the SPARC family genes also can be used, in which event they are preferably not used in the chromosome naturally encoding them unless modified by a process that substantially changes that chromosome. Such substantially changed chromosomes can include chromosomes transfected and altered by a retroviral vector or similar process. Alternatively, such substantially changed chromosomes can comprise an artificial chromosome such as a HAC, YAC, or BAC.

In addition, the nucleic acid molecules described herein may be operatively linked to enhancers to facilitate transcription. Enhancers are cis-acting elements of DNA that stimulate the transcription of adjacent genes. Examples of enhancers which confer a high level of transcription on linked genes in a number of different cell types from many species include, without limitation, the enhancers from SV40 and the RSV-LTR. Such enhancers can be combined with other enhancers which have cell type-specific effects, or any enhancer may be used alone.

To optimize protein production the inventive nucleic acid molecule can further comprise a polyadenylation site following the coding region of the nucleic acid molecule. Also, preferably all the proper transcription signals (and translation signals, where appropriate) will be correctly arranged such that the exogenous nucleic acid will be properly expressed in the cells into which it is introduced. If desired, the exogenous nucleic acid also can incorporate splice sites (i.e., splice acceptor and splice donor sites) to facilitate mRNA production while maintaining an inframe, full length transcript. Moreover, the inventive nucleic acid molecules can further comprise the appropriate sequences for processing, secretion, intracellular localization, and the like.

The nucleic acid molecules may be inserted into any suitable vector. Suitable vectors include, without limitation, viral vectors. Suitable viral vectors include, without limitation, retroviral vectors, alphaviral, vaccinial, adenoviral, adenoassociated viral, herpes viral, and fowl pox viral vectors. The vectors preferably have a native or engineered capacity to transform eukaryotic cells, e.g., 293 cells. Additionally, the vectors useful in the context of the invention can be "naked" nucleic acid vectors (i.e., vectors having little or no proteins, sµgars, and/or lipids encapsulating them) such as plasmids or episomes, or the vectors can be complexed with other molecules. Other molecules that can be suitably combined with the inventive nucleic acids include without limitation viral coats, cationic lipids, liposomes, polyamines, gold particles, and targeting moieties such as ligands, receptors, or antibodies that target cellular molecules.

The nucleic acid molecules described herein may be transformed into any suitable cell, typically a eukaryotic cell, such as, e.g., HEK, 293, or BHK, desirably resulting in the expression of a SPARC polypeptide such as, e.g., polypeptide comprising of SEQ ID NO: 2 or a variant thereof as described herein. The cell can be cultured to provide for the expression of the nucleic acid molecule and, therefore, the production of the SPARC polypeptide such as, e.g., a polypeptide comprising the amino acid sequence of SEQ ID NO: 2 or a variant thereof as described herein.

Therefore, the invention provides for a cell transformed or transfected with an inventive nucleic acid molecule described herein. Means of transforming, or transfecting, cells with exogenous DNA molecules are well known in the art. For example, without limitation, a DNA molecule is introduced into a cell using standard transformation or transfection techniques well known in the art such as calcium-phosphate or DEAE-dextran-mediated transfection, protoblast fusion, electroporation, liposomes and direct microinjection (see, e.g., Sambrook & Russell, Molecular Cloning: A Laboratory Manual, Cold Spring Harbor Laboratory Press, New York (2001), pp. 1.1-1.162, 15.1-15.53, 16.1-16.54). A widely used method for transformation is transfection mediated by either calcium phosphate or DEAE-dextran. Depending on the cell type, up to 20% of a population of cultured cells can be transfected at any one time.

Another example of a transformation method is the protoplast fusion method, protoplasts derived from bacteria carrying high numbers of copies of a plasmid of interest are mixed directly with cultured mammalian cells. After fusion of the cell membranes (usually with polyethylene glycol), the contents of the bacteria are delivered into the cytoplasm of the mammalian cells, and the plasmid DNA is transferred to the nucleus. Protoplast fusion is not as efficient as transfection for many of the cell lines that are commonly used for transient expression assays, but it is useful for cell lines in which endocytosis of DNA occurs inefficiently. Protoplast fusion frequently yields multiple copies of the plasmid DNA randomly integrated into the host chromosome.

Electroporation, the application of brief, high-voltage electric pulses to a variety of mammalian and plant cells leads to the formation of nanometer-sized pores in the plasma membrane. DNA is taken directly into the cell cytoplasm either throµgh these pores or as a consequence of the redistribution of membrane components that accompanies closure of the pores. Electroporation can be extremely efficient and can be used both for transient expression of clones genes and for establishment of cell lines that carry integrated copies of the gene of interest.

Liposome transformation involves encapsulation of DNA and RNA within liposomes, followed by fusion of the liposomes with the cell membrane. In addition, DNA that is coated with a synthetic cationic lipid can be introduced into cells by fusion. Alternatively, linear and/or branched polyethylenimine (PEI) can be used in transfection.

Direct microinjection of a DNA molecule into nuclei has the advantage of not exposing the DNA molecule to cellular compartments such as low-pH endosomes. Microinjection is, therefore, used primarily as a method to establish lines of cells that carry integrated copies of the DNA of interest.

Such techniques can be used for both stable and transient tranformation of eukaryotic cells. The isolation of stably transformed cells requires the introduction of a selectable marker in conjunction with the transformation with the gene of interest. Such selectable markers include genes which confer resistance to neomycin as well as the HPRT gene in HPRT negative cells. Selection can require prolonged culture in selection media, at least for about 2-7 days, preferable for at least about 1-5 weeks (see, e.g., Sambrook & Russell, Molecular Cloning: A Laboratory Manual, Cold Spring Harbor Laboratory Press, New York (2001), pp. 16.1-16.54).

Nucleic acid sequences for use in the present invention may also be produced in part or in total by chemical synthesis, e.g. by the phosphoramidite method described by Beaucage, et al. (Tetra. Letts. 22: 1859-1862 (1987)), or the triester method (Matteucci et al., J. Am. Chem. Soc. 103: 3185 (1981)), which may be performed on commercial automated oligonucleotide synthesizers. A double-stranded fragment may be obtained from the single-stranded product of chemical synthesis either by synthesizing the complementary strand and annealing the strand together under appropriate conditions, or by synthesizing the complementary strand using DNA polymerase with an appropriate primer sequence.

SPARC polypeptide may be made by any suitable recombinant method comprising a vector engineered to overexpress exogenous SPARC. Any suitable cells, including bacteria, yeast, insect or mammalian cells can be transfected to express SPARC. African Green Monkey-293 are the preferred mammalian cells for exogenous SPARC expression. While any suitable culture system can be used, a hollow fiber cell system is a preferred culture system for SPARC production.

Since SPARC is secreted it can be isolated from the media as follows: (a) conditioned media collected twice daily from bioreactor (e.g., 100ml /large cartridge); (b) media centrifµged and filtered throµgh 0.22 micron filter; ph of filtrate is adjusted to 7.8 before loading on the affinity column. Surprisingly, serum has been found to protect secreted SPARC from degradation, with 3% serum a sufficient concentration to afford this protection.

Any suitable purification method can be used. For example, a Ni-affinity column is ideal for histidine tagged SPARC. Firsts the Column is equilibrated with 50mM Na-P, 0.5M NaCl, pH 7.8. The sample is then loaded and washed the column with same buffer until the baseline. The column is washed with same buffer, but with pH 6.0 until baseline. This is followed washing with same buffer, but with pH 5.3 until baseline Bound protein is eluted with immidazole gradient as follows: (a) the column is washed with 2 column volume of buffer A (1X PBS, 300mM NaCl, pH 7.9), (b) a 10% gradient to buffer B (1X PBS, 300mM NaCl, 500mM Immidazole, pH 7.9) is applied for up to 10 column volumes, (c) a gradient to 100% buffer B is used to elute any proteins bound. Peak fractions of immidazole gradient were analyzed by Western blot

Mono-Q ion-exchange chromatography is, optionally, used for further purification. SPARC containing fractions of Ni-column are pooled and then concentrated and buffer changed using Amicon centricon to 20mM MOPS, 200mM LiCl2, pH 6.5. This sample is loaded onto Mono-Q column which is pre-equilibrated with same buffer. Bound protein is eluted with linear gradient of 20mM Mops, 200mM LiCl2, pH 6.5

After each column chromatography, SPARC containing fractions are analyzed by Western blot. Pooled fractions containing SPARC are further concentrated and buffer changed using Amicon centricon (i.e. after Ni-column, buffer is changed for Mono-Q column, and PBS after Mono-Q purification). This purified protein is analyzed by SDS-PAGE and bands were scanned for purity. Endotoxin level is determined by a colorimetric method.

In certain bacterial embodiments, when expressing and purifying a SPARC polypeptide, techniques for improving protein solubility are employed to prevent the formation of bacterial inclusion body (which are insoluble fractions), and therefore obtaining large quantities of the polypeptide. SPARC accumulated in inclusion bodies is an inactive-type SPARC not retaining its physiological activities.

Solubility of a purified SPARC polypeptide can be improved by methods known in the art. For example, solubility may also be improved by expressing a functional fragment, but not the full length SPARC polypeptide. In addition, to increase the solubility of an expressed protein (e.g., in *E. coli*), one can reduce the rate of protein synthesis by lowering the growth temperature, using a weaker promoter, using a lower copy number plasmid, lowering the inducer concentration, changing the growth medium as described in Georgiou & Valax (Current Opinion Biotechnol. 7:190-197 (1996)). This decreases the rate of protein synthesis and usually more soluble protein is obtained. One can also add prosthetic groups or co-factors which are essential for proper folding or for protein stability, or add buffer to control pH fluctuation in the medium during growth, or add 1% glucose to repress induction of the lac promoter by lactose, which is present in most rich media (such as LB, 2xYT). Polyols (e.g., sorbitol) and sucrose may also be added to the media because the increase in osmotic pressure caused by these additions leads to the accumulation of osmoprotectants in the cell, which stabilize the native protein structure. Ethanol, low molecular weight thiols and disulfides, and NaCl may be added. In addition, chaperones and/or foldases may be co-expressed with the desired polypeptide. Molecular chaperones promote the proper isomerization and cellular targeting by transiently interacting with folding intermediates. E. coli chaperone systems include but, are not limited to: GroES-GroEL, DnaK-DnaJ-GrpE, CIpB.

Foldases accelerate rate-limiting steps along the folding pathway. Three types of foldases play an important role: peptidyl prolyl cis/trans isomerases (PPI's), disulfide oxidoreductase (DsbA) and disulfide isomerase (DsbC), protein disulfide isomerase (PDI) which is an eukaryotic protein that catalyzes both protein cysteine oxidation and disulfide bond isomerization. Co-expression of one or more of these proteins with the target protein could lead to higher levels of soluble target protein.

A SPARC polypeptide can be produced as a fusion protein in order to improve its solubility and production. The fusion protein comprises a SPARC polypeptide and a second polypeptide fused together in frame. The second polypeptide may be a fusion partner known in the art to improve the solubility of the polypeptide to which it is fused, for example, polyhistidine tag, NusA, bacterioferritin (BFR), GrpE, thioredoxin (TRX) and glutathione-S-transferase (GST). Novagen Inc. (Madison, Wis.) provides the pET 43.1 vector series which permit the formation of a NusA-target fusion. DsbA and DsbC have also shown positive effects on expression levels when used as a fusion partner, therefore can be used to fuse with a SPARC polypeptide for achieving higher solubility.

In one embodiment, a SPARC polypeptide is produced as a fusion polypeptide comprising the SPARC polypeptide and a fusion partner thioredoxin, as described in U.S. Pat. NO:6,387,664, hereby incorporated by reference in its entirety. The thioredoxin-SPARC fusion can be produced in E. coli as an easy-to-formulate, soluble protein in a large quantity without losing the physiological activities. Although U.S. Pat. NO:6,387,664 provides a fusion SPARC protein with SPARC fused to the C-terminus of thioredoxin, it is understood, for the purpose of the present invention, a SPARC polypeptide can be fused either to the N-tenninus or the C-terminus of a second polypeptide, so long as its sensitizing function is retained.

In addition to increase solubility, a fusion protein comprising a SPARC polypeptide can be constructed for the easy detection of the expression of the SPARC polypeptide in a cell. In one embodiment, the second polypeptide which fused to the SPARC polypeptide is a reporter polypeptide. The reporter polypeptide, when served for such detection purpose, does not have to be fused with the SPARC polypeptide. It may be encoded by the same polynucleotide (e.g., a vector) which also encodes the SPARC polypeptide and be co-introduced and co-expressed in a target cell.

Preferably, the reporter polypeptide used in the invention is an autofluorescent protein (e.g., GFP, EGFP). Autofluorescent proteins provide a ready assay for identification of expression of a polynucleotide (and the polypeptide product) of interest. Because the activity of the reporter polypeptide (and by inference its expression level) can be monitored quantitatively using a flow sorter, it is simple to assay many independent transfectants either sequentially or in bulk population. Cells with the best expression can then be screened for or selected from the population. This is useful when selecting a recombinant cell comprising a SPARC polypeptide or polynucleotide for sensitizing treatment according to the present invention.

The invention provides for SPARC molecules, including SPARC polypeptides and proteins conjugated to polyethylene glycol (PEG). PEG conjµgation can increase the circulating half-life of a protein, reduce the protein's immunogenicity and antigenicity, and improve the bioactivity. Any suitable method of conjµgation can be used, including but not limited to, e.g., reacting methoxy-PEG with a SPARC protein's available amino groups or other reactive sites such as, e.g., histidines or cysteines. In addition, recombinant DNA approaches may be used to add amino acids with PEG-reactive groups to the inventive SPARC molecules. PEG can be processed prior to reacting it with the inventive SPARC protein, e.g., linker groups may be added to the PEG. Further, releasible and hybrid PEG-ylation strategies may be used in accordance with the invention, such as, e.g., the PEG-ylation of SPARC such that the PEG molecules added to certain sites in the SPARC molecule are released in vivo. Such PEG conjµgation methods are known in the art (See, e.g., Greenwald et al., Adv. Drug Delivery Rev. 55:217-250 (2003)).

In addition, the invention provides for SPARC fusion proteins, including, for example without limitation, SPARC sequences are fused upstream or downstream of diagnostically useful protein domains (such as hapten, GFP), immunologically active protein domains (e.g., TF or TNF) or toxin domains.

In addition, the invention provides for a method of treating a tumor or other proliferative disease in a mammal with a chemotherapeutic agent or other anticancer agent comprising: (a) isolating a biological sample from the mammal, (b) detecting the expression of SPARC protein or RNA in the biological sample, (c) quantifying the amount of SPARC protein or RNA in the biological sample, (d) determining if the SPARC protein or RNA is present at a level indicating the use of the chemotherapeutic agent or other anticancer agent, and (e), if, based on the SPARC protein or RNA level, it is indicated, administering a therapeutically effective amount of the chemotherapeutic agent or other anticancer agent.

II. Diagnostic Aspects

By "predicting the response of a human or other mammalian tumor or other proliferative disease to a chemotherapeutic agent" it is meant making a judgment, based on test results combined with clinical experience, regarding the likelihood of a response before administering the chemotherapeutic agent. By "determining the response of a human or other mammalian tumor to a chemotherapeutic agent" it is meant making a judgment, based on test results combined with clinical experience, regarding the likelihood of a response after administering the chemotherapeutic agent but, before the response can be determined clinically or by conventional laboratory or imagining studies known to those of ordinary skill in the medical arts. By tumor it is meant a clonal proliferation of cells which may or may not have malignant properties (e.g., without limitation, the ability to induce angiogenesis, invade, be free of contact or ischemia or inhibited growth, metastesize or have impaired DNA repair.)

As used herein, the terms "resistant" or "resistance to a chemotherapeutic or other anticancer agent" refers to an acquired or natural resistance of a cancer sample or a mammal to a therapy, i.e., being nonresponsive to or having reduced or limited response to the therapeutic treatment, e. g. , having a reduced response to a therapeutic treatment by 25% or more. Further, resistance can also be indicated by a reduced response of, for example, 30%, 40%, 50%, 60%, 70%, 80%, or more, to 2-fold, 3-fold, 4-fold, 5-fold, 10-fold, 15-fold, 20-fold or more. The reduction in response is measured by comparing with the same cancer sample or mammal before the resistance is acquired, or by comparing with a different cancer sample or a mammal who is known to have no resistance to the therapeutic treatment. As used herein, the terms "sensitive" or "sensitive to a chemotherapeutic or other anticancer agent" refers to the absence of resistance.

The invention provides methods for predicting or determining the response of a mammalian tumor or other proliferative disease to a chemotherapeutic agent or other anticancer agent, the method comprising the steps of (a) isolating a biological sample from the mammal, (b) detecting the expression of SPARC protein or RNA in the biological sample, and (c) quantifying the amount of SPARC protein in the biological sample. The method can be used in accordance with a further and related aspect of the invention wherein SPARC protein or RNA is overexpressed or underexpressed in the tumor relative to the corresponding normal tissue. By "corresponding normal tissue" it is meant the tissue, in the absence of tumor, in which the primary tumor develops or the tissue which, in the absence of tumor, contains the type of cells or stem cells which have been transformed or mutated so as to become the neoplastic cells of the tumor. The invention provides for embodiments wherein the biological sample is isolated from the tumor (or tissue involved with a proliferative disease) or from a bodily fluid, such as, e.g., cerebrospinal fluid, blood, plasma, serum or urine. The invention further provides a method for predicting or determining the response of a mammalian tumor or other proliferative disease to a chemotherapeutic agent or other anticancer agent, wherein the mammal is a human.

In addition, the invention provides a method of treating a tumor or other proliferative disease in a mammal with a chemotherapeutic agent or other anticancer agent comprising (a) isolating a biological sample from the mammal, such as, e.g., a human, (b) detecting the expression of SPARC protein or RNA in the biological sample, (c) quantifying the amount of SPARC protein or RNA in the biological sample, (d) determining if the SPARC protein or RNA is present at a level indicating that a chemotherapeutic agent or other anticancer agent should be administered, and (e), if, based on the SPARC protein or RNA level, it is indicated, administering a therapeutically effective amount of the chemotherapeutic agent or other anticancer agent. In particular, the invention provides methods of treating a mammalian tumor comprising combination therapy with SPARC and albumin-bound paclitaxel.

Further, the invention provides a kit for predicting the response of a mammalian tumor, such as, e.g., a human tumor, or other proliferative disease to a chemotherapeutic agent or other anticancer agent, comprising a means for the isolation of protein from the tumor, a SPARC protein detection and quantification means, control proteins, and rules for predicting the response of the tumor. The invention also provides a kit for predicting the response of a mammalian tumor or other proliferative disease to a chemotherapeutic agent or other anticancer agent comprising a means for the isolation of RNA from the tumor, a SPARC RNA detection and quantification means, control RNAs, and rules for predicting the response of the tumor based on the level of SPARC RNA in the tumor.

The invention also provides a method for predicting or determining the response of a mammalian tumor to a chemotherapeutic agent, as well as a method for treating a mammalian tumor with a chemotherapeutic agent, wherein the chemotherapeutic agent is, e.g., epothilone, docetaxel, paclitaxel (such as Abraxane^{®}) or combinations thereof. The invention further provides for embodiments wherein the prediction of the response of a mammalian tumor to a chemotherapeutic agent is positively or negatively correlated with SPARC levels.

Further, the invention provides a method for delivering a chemotherapeutic agent to a tumor in the mammal, wherein the method comprises administering to a mammal a therapeutically effective amount of a pharmaceutical composition, wherein the pharmaceutical composition comprises a chemotherapeutic agent coupled to a SPARC protein capable of binding albumin and a pharmaceutically acceptable carrier. The inventive compositions may comprise small molecules, large molecules or proteins.

By "determining if the SPARC protein or RNA is present at a level indicating the use of the chemotherapeutic agent" it is meant that the quantified level of SPARC protein or RNA is present in the specimen from the mammal with a tumor is high enoµgh, based on a comparison historical correlation data of SPARC level and treatment response, to indicate that the tumor can be reasonably expected to respond to the chemotherapeutic agent. By "indicating" or "indicated" it meant that, in view of the SPARC level and based on reasonable medical judgment, the chemotherapeutic agent should be used. For example, without limitation, a biopsy of a tumor can be prepared for immunohistology with anti-SPARC antibodies by preparing a thin section of the biopsy on a microscope slide. Then, the biopsy slide is stained using an anti-SPARC immunohistological protocol (see, e.g., Sweetwyne et al., J. Histochem. Cytochem. 52(6):723-33 (2004); Tai et al., J. Clin. Invest. 115(6):1492-502 (2005)) simultaneously with control slides containing sections of biopsies with known SPARC levels from other tumors sensitive to and resistant to the chemotherapeutic agent considered for use. It is a common practice in the art to grade the intensity of immunohistological staining using light microscopy. The ordinarily skilled artisan (e.g., a pathologist) can, based on comparison with the staining of the control slides, assign a staining grade (e.g., 0, 1+, 2+, 3+, 4+) to the tumor biopsy. Treatment with the chemotherapeutic agent can be "indicated" if the staining of the tumor biopsy is graded at, e.g., 3+ or 4+. Such comparisons and assignments of staining grades are well within the skill of the ordinarily skilled medical artisan (e.g., physician, pathologist, oncologist, veterinarian) treating mammals with tumors.

The methods call for a biological sample which can be isolated from the tumor or tissues involved with a proliferative disease by any suitable procedure including, without limitation, resection, biopsy, aspiration, venupuncture or combinations thereof. Alternatively, the methods call for a biological sample which can be from a bodily fluid, such as, e.g., cerebrospinal fluid, blood, plasma, serum, and urine. In addition, control or reference biological samples including tumor and bodily fluid materials can be obtained from normal tissues of the same mammal, other individuals free of tumor or proliferative disease or from other tumors with known SPARC levels and known to be sensitive to or resistant to a given chemotherapeutic agent. Additionally, the methods can be practiced wherein the mammal suffering from the tumor or proliferative disease is a human.

Further, the invention provides for a kit for predicting the response of a mammalian tumor or other proliferative disease to a chemotherapeutic agent or other anticancer agent, comprising a means for the isolation of protein from the tumor, a SPARC protein detection and quantification means, control proteins, and rules for predicting the response of the tumor. The invention also provides for a kit for predicting the response of a mammalian tumor or other proliferative disease to a chemotherapeutic agent or other anticancer agent, comprising a means for the isolation of RNA from the tumor, a SPARC RNA detection and quantification means, control RNAs, and rules for predicting the response of the tumor based on the level of SPARC RNA in tumor. For example, the SPARC protein or RNA in a tumor biopsy can be "isolated" by placing a thin section of the tumor biopsy on a microscope slide. Any SPARC protein or RNA present can then be detected and quantified by immunohistological staining with an anti-SPARC antibody (see, e.g., Sweetwyne et al., J. Histochem. Cytochem. 52(6):723-33 (2004); Tai et al., J. Clin. Invest. 115(6):1492-502 (2005)) or in situ hybridization using a nucleic acid probe complementary to SPARC RNA (see, e.g., Thomas et al., Clin. Can. Res. 6:1140-49 (2000)). At the same time positive and negative control slides would be stained for SPARC protein or RNA. The ordinarily skilled artisan can readily use light microscopy to grade the staining intensity of the SPARC in the tumor biopsy (e.g., 0, 1+, 2+, 3+, 4+). The inventive kit also comprises rules for predicting the response of the tumor based on the level of SPARC protein or RNA in tumor, such as, e.g., "treatment with the chemotherapeutic agent is indicated if the staining of the tumor biopsy is graded at, e.g., 3+ or 4+" or "tumors with 3+ or 4+ staining have a high response rate." The specific rules relating to a particular embodiment of the inventive kits can readily be generated by performing retrospective or prospective correlation studies which are routine in the art and which would not require undue experimentation.

By "quantification" as used herein it is meant determining the amount or concentration present. The invention provides for a method of quantifying the level of SPARC protein or RNA wherein SPARC protein or RNA is overexpressed or underexpressed in the tumor relative to normal tissues, including but, not limited to, the level found in the corresponding normal tissue of origin of the tumor. Alternatively, The invention provides for a method of quantifying the level of SPARC protein or RNA wherein SPARC protein or RNA is overexpressed or underexpressed in the tumor relative to other tumors, including but not limited to, tumors of the same tissue or histology. Further, The invention provides for a method of quantifying the level of SPARC protein or RNA wherein SPARC protein or RNA is overexpressed or underexpressed in the tumor relative to other tumors, including but, not limited to, tumors which are sensitive to or resistant to a chemotherapeutic agent or combination of chemotherapeutic agents. By overexpressed or underexpressed it is meant that the levels of SPARC protein or RNA differs between the two specimens or samples by at least about 5%. Further, it is desirable that the difference between the two specimens or samples is at least about 10%, more preferably at least about 20%, more preferably at least about 50%, more preferably at least about 100%, more preferably at least about 3 fold, more preferably at least about 5 fold, and most preferably at least about 10 fold.

The invention provides for a method of quantifying the level of SPARC protein or RNA wherein SPARC protein or RNA is overexpressed or underexpressed in the test biological fluid relative to corresponding fluid from a tumor-free patient. Alternatively, The invention provides for a method of quantifying the level of SPARC protein or RNA wherein SPARC protein or RNA is overexpressed or underexpressed in the test biological fluid relative to corresponding fluid from a another patient with a tumor, including but not limited to, tumors which are sensitive to or resistant to a chemotherapeutic agent or combination of chemotherapeutic agents. By overexpressed or underexpressed it is meant that the levels of SPARC protein or RNA differs in two specimens by at least about 5%. Further, it is desirable that a difference of at least about 10% is present, preferably at least about 20%, more preferably by at least about 50%, more preferably by at least about 100%, more preferably at least about 3 fold, more preferably at least about 5 fold, and most preferably at least about 10 fold.

The invention provides methods of predicting or determining a tumor's response to a chemotherapeutic agent or other anticancer agents, methods of treating a tumor, and kits for predicting the response of a mammalian tumor to a chemotherapeutic agent or other anticancer agent, wherein the tumor is selected from the group consisting of oral cavity tumors, pharyngeal tumors, digestive system tumors, the respiratory system tumors, bone tumors, cartilaginous tumors, bone metastases, sarcomas, skin tumors, melanoma, breast tumors, the genital system tumors, urinary tract tumors, orbital tumors, brain and central nervous system tumors, gliomas, endocrine system tumors, thyroid tumors, esophageal tumors, gastric tumors, small intestinal tumors, colonic tumors, rectal tumors, anal tumors, liver tumors, gall bladder tumors, pancreatic tumors, laryngeal tumors, tumors of the lung, bronchial tumors, non-small cell lung carcinoma, small cell lung carcinoma, uterine cervical tumors, uterine corpus tumors, ovarian tumors, vulvar tumors, vaginal tumors, prostate tumors, prostatic carcinoma, testicular tumors, tumors of the penis, urinary bladder tumors, tumors of the kidney, tumors of the renal pelvis,tumors of the ureter, head and neck tumors, parathyroid cancer, Hodgkin's disease, Non-Hodgkin's lymphoma, multiple myeloma, leukemia, acute lymphocytic leukemia, chronic lymphocytic leukemia, acute myeloid leukemia, chronic myeloid leukemia. In addition, the invention provides for method of predicting or determining a tumor's response to a chemotherapeutic agent, methods of treating a tumor, and kits for predicting the response of a mammalian tumor to a chemotherapeutic agent, wherein the tumor is a sarcoma, adenocarcinoma, squamous cell carcinoma, large cell carcinoma, small cell carcinoma, basal cell carcinoma, clear cell carcinoma, oncytoma or combinations thereof. Further, the invention provides for method of predicting or determining a tumor's response to a chemotherapeutic agent, methods of treating a tumor, and kits for predicting the response of a mammalian tumor to a chemotherapeutic agent, wherein the tumor is a benign tumor or a malignant tumor. Yet further, the invention provides for method of predicting or determining a proliferative disease's response to a chemotherapeutic agent or treating a proliferative disease, including but, not limited to, where the proliferative diseases is, e.g., benign prostatic hyperplasia, endometriosis, endometrial hyperplasia, atherosclerosis, psoriasis or a proliferative renal glomerulopathy. The invention provides for embodiments wherein the tumor is in mammal including but, not limited to, where the mammal is a human.

Any suitable biological sample can be isolated from the mammal in the context of the inventive method and used for polypeptide and/or RNA detection and quantification. Preferably, the biological sample is isolated from the tumor, such as by a tumor biopsy. The biological sample is isolated from the mammal using methods known in the art. Alternatively, the biological sample can be isolated from a bodily fluid of the mammal, including, for example, cerebrospinal fluid, blood, plasma, serum, or urine. In particular, many protein purification techniques are known in the art (see, e.g., Harlow & Lane, Antibodies, A Laboratory Manual, Cold Spring Harbor, pp. 421-696 (1988)).

Any suitable method for the detection and quantification of a SPARC protein can be used in accordance with the invention including, but not limited to, the use of anti-SPARC antibodies (e.g., Western blot, ELISA) (see, e.g., Sweetwyne et al., J. Histochem. Cytochem. 52(6):723-33 (2004); Tai et al., J. Clin. Invest. 115(6):1492-502 (2005)), the use of SPARC-specific binding proteins (e.g., radiolabel SPARC ligands, ELISA-like assays), two-dimensional electrophoresis, mass spectroscopy or combinations thereof (see, e.g., Nedelkov D et al., Proc. Natl. Acad. Sci. U.S.A. 102(31):10852-7 (2005); Chen et al., Proc. Natl. Acad. Sci. U.S.A. 101 (49):17039-44(2004)). Further, immunohistochemistry can be used for the isolation, detection and quantification of SPARC protein in a sample (see, e.g., Sweetwyne et al., J. Histochem. Cytochem. 52(6):723-33 (2004); Tai et al., J. Clin. Invest. 115(6):1492-502 (2005)).

The invention provides for a method wherein the SPARC RNA is detected and quantified. Numerous methods are known in the art to isolate RNA, such as the ones described by Chomczynski (U.S. Pat. NO:5,945,515) or by DiMartino et al. (Leukemia 20(3):426-32 (2006)). Alternatively, RNA can be isolated in a form suitable for detection and quantification in accordance with the invention by the preparation of a microscope slide containing a tissue section (see, e.g., Thomas et al., Clin. Can. Res. 6:1140-49 (2000)). SPARC RNA can be detected and quantified by any suitable method known in the art including but, not limited to, in situ hybridization (see, e.g., Thomas et al., Clin. Can. Res. 6:1140-49 (2000)), Northern blot (see e.g., Wrana et al., Eur. J. Biochem. 197:519-28 (1991)), real-time RT-PCR (see, e.g., DiMartino et al., Leukemia 20(3):426-32 (2006)), Real-time nucleic acid sequence-based amplification (see, e.g., Landry et al., J. Clin. Microbiol. 43(7):3136-9 (2005)), microarray analysis (see, e.g., Tai et al., J. Clin. Invest. 115(6):1492-502 (2005); DiMartino et al., Leukemia 20(3):426-32 (2006)) and combinations thereof.

The invention also provides a method for predicting or determining the response of a human or other mammalian tumor or other proliferative disease to a chemotherapeutic agent or other anticancer agents wherein the response of a mammalian tumor to a chemotherapeutic agent is positively or negatively correlated with SPARC levels. By "correlated with SPARC levels" it is meant, e.g., that a mutual or reciprocal relation between the tumor's response to a given chemotherapeutic agent and the level of SPARC protein or RNA detected. That is, the quality, degree, magnitude, or level of the tumor response varies with the level of the level of SPARC protein or RNA detected. A "positive correlation" is present when the quality, degree, magnitude, or level of the tumor response increases as the level of SPARC protein or RNA detected increases. A "negative correlation" is present when the quality, degree, magnitude, or level of the tumor response decreases as the level of SPARC protein or RNA detected increases. The relation between the level the tumor response and the level of SPARC protein or RNA detected can take on the form of or approximate a step-function, linear-function or logarithmic function. By "correlating" it is meant establishing a correlation or considering the impact of a known correlation.

In addition, the invention also provides a method for predicting or determining the response of a human or other mammalian tumor or other proliferative disease to a chemotherapeutic agent or other anticancer agents by comparing the level of SPARC protein or RNA detected to that detected in a known reference sample. Such a reference sample can be from, for example, a normal tissue or bodily fluid. Alternatively, the reference sample can be a tumor with a known SPARC level, response, sensitivity or resistance to a given chemotherapeutic agent or other anticancer agents or combinations thereof

Further, the predicted response can be characterized as effective or as not effective such that a given chemotherapeutic agent would be used or an alternative chemotherapeutic agent would be used. As such, the predicted response can be characterized as a ratio of the response resulting from the use of one chemotherapeutic agent versus the use of another chemotherapeutic agent, e.g., the ratio of the response produced by Abraxane® to that produced by Taxotere®.

Accordingly, the invention provides for a kit for predicting the response of a mammalian tumor or other proliferative disease to a chemotherapeutic agent or other anticancer agents, comprising a means for the isolation of protein or RNA from the tumor, a SPARC protein or RNA detection and quantification means, control proteins or RNAs, and rules for predicting the response of the tumor. Such a kit can, for example without limitation, be used to predict the response of a breast, ovarian or head and neck carcinoma to a chemotherapeutic agent comprising nanoparticles of albumin-bound paclitaxel. Suitable means for isolating protein or RNA and a SPARC protein or RNA detection and quantification have been described herein. Suitable control proteins or RNAs should include positive controls such as, e.g., tumor material or biological fluid from a tumor bearing mammal or isolated protein or RNA from tumor material or from a biological fluid harvested from a tumor bearing mammal. Suitable control proteins or RNAs include negative controls such as, e.g., normal tissue or biological fluid from a mammal free of tumor or isolated protein or RNA from normal tissue or biological fluid harvested from a mammal free of tumor. Controls in the kit can also include materials use to establish standard curves for quantification of SPARC protein or RNA or material from sensitive and resistant tumors. The kits of the invention can also comprising a means for determining the Her2 status of the tumor.

The inventive kits would further comprise rules for predicting the response of the tumor. Such rules would base the prediction of response to a given chemotherapeutic agent on the level of SPARC protein or RNA detected as described herein in relation to the methods of predicting or determining a response to chemotherapeutic agent. For example, a particular level of SPARC protein or RNA, based on past experience, can indicate that a chemotherapeutic agent should be used. It is within the skill of the ordinarily skilled artisan to generate, without undue experimentation, adequate data (by prospective studies, retrospective studies or a combination thereof) to determine the level of SPARC protein or RNA predictive of response to a given chemotherapeutic agent.

The SPARC protein is responsible for the accumulation of albumin in certain human tumors. As albumin is the major carrier of chemotherapeutic drugs, the expression level of SPARC is indicative of the amount of chemotherapeutic drug that penetrates and is retained by the tumor. Therefore, the expression level of SPARC is predictive of the responsiveness of the tumor to chemotherapy.

Any suitable biological sample can be isolated from the mammal of interest in the context of the inventive method. Preferably, the biological sample is isolated from the tumor, such as by a tumor biopsy. Alternatively, the biological sample can be isolated from a bodily fluid of the mammal, including, for example, cerebrospinal fluid, blood, plasma, serum, or urine. Techniques and methods for the isolation of biological samples are known to those in the art.

The types of tumor to be detected, whose response to chemotherapy is to be predicted or determined, which can be treated in accordance with the invention are generally those found in humans and other mammals. The tumors can be the result of inoculation as well, such as in laboratory animals. Many types and forms of tumors are encountered in human and other animal conditions, and there is no intention to limit the application of the methods of the present to any particular tumor type or variety. Tumors, as is known, include an abnormal mass of tissue that results from uncontrolled and progressive cell division, and is also typically known as a "neoplasm." The inventive methods are useful for tumor cells and associated stromal cells, solid tumors and tumors associated with soft tissue, such as, soft tissue sarcoma, for example, in a human. The tumor or cancer can be located in the oral cavity and pharynx, the digestive system, the respiratory system, bones and joints (e.g., bony metastases), soft tissue, the skin (e.g., melanoma), breast, the genital system, the urinary system, the eye and orbit, the brain and central nervous system (e.g., glioma), or the endocrine system (e.g., thyroid) and is not necessarily limited to the primary tumor or cancer. Tissues associated with the oral cavity include, but are not limited to, the tongue and tissues of the mouth. Cancer can arise in tissues of the digestive system including, for example, the esophagus, stomach, small intestine, colon, rectum, anus, liver, gall bladder, and pancreas. Cancers of the respiratory system can affect the larynx, lung, and bronchus and include, for example, non-small cell lung carcinoma. Tumors can arise in the uterine cervix, uterine corpus, ovary vulva, vagina, prostate, testis, and penis, which make up the male and female genital systems, and the urinary bladder, kidney, renal pelvis, and ureter, which comprise the urinary system. The tumor or cancer can be located in the head and/or neck (e.g., laryngeal cancer and parathyroid cancer). The tumor or cancer also can be located in the hematopoietic system or lymphoid system, and include, for example, lymphoma (e.g., Hodgkin's disease and Non-Hodgkin's lymphoma), multiple myeloma, or leukemia (e.g., acute lymphocytic leukemia, chronic lymphocytic leukemia, acute myeloid leukemia, chronic myeloid leukemia, and the like). Preferably, the tumor is located in the bladder, liver, ovary, kidney, gut, brain, or breast.

III. Targeting Embodiments

The invention also provides a method for delivering a chemotherapeutic agent to a tumor in a human or other mammal. The method comprises administering to a human or other mammal a therapeutically effective amount of a delivery agent, such as a pharmaceutical composition, wherein the delivery agent (e.g., pharmaceutical composition) comprises the chemotherapeutic agent coupled to a SPARC polypeptide. Pharmaceutical compositions preferably include the chemotherapeutic agent coupled to the SPARC recognition group and a pharmaceutically acceptable carrier. Descriptions of the chemotherapeutic agent, tumor, mammal, and components thereof, set forth herein in connection with other embodiments of the invention also are applicable to those same aspects of the aforesaid method of delivering a chemotherapeutic agent to a tumor.

In other embodiments, the invention provides a method for delivering a pharmaceutically active agent by way of a SPARC polypeptide to a site of disease that expressed a SPARC binding moiety. Such diseases include abnormal conditions of proliferation, tissue remodeling, hyperplasia, and exaggerated wound healing in bodily tissue (e.g., soft tissue, connective tissue, bone, solid organs, blood vessel and the like). Examples of diseases that are treatable or may be diagnosed by administering a pharmaceutical composition comprising a therapeutic agent coupled to a compound or ligand capable of binding a SPARC protein, or another albumin-binding protein, include cancer, diabetic or other retinopathy, inflammation, arthritis, restenosis in blood vessels, artificial blood vessel grafts, or intravascular devices, and the like. Descriptions of the pharmaceutically active agent, tumor, mammal, and components thereof, set forth herein in connection with other embodiments of the invention also are applicable to those same aspects of the aforesaid method of delivering a pharmaceutically active agent.

The invention also provides a method for delivering a chemotherapeutic agent to a tumor in a mammal. The method comprises administering to a mammal a therapeutically effective amount of a pharmaceutical composition, wherein the pharmaceutical composition comprises the chemotherapeutic agent coupled to a SPARC protein capable of binding albumin and a pharmaceutically acceptable carrier.

Methods for coupling or conjµgation of suitable therapeutics, chemotherapeutics, radionuclides, polypeptides, and the like to antibodies or fragments thereof are well described in the art. For example, The invention provides for SPARC polypeptide such as, e.g., SPARC- radioinuclide, SPARC-drug, SPARC-immunomodulator or SPARC-toxin conjµgates. Any suitable method can be used in accordance with the invention to form the SPARC conjµgates. For example, without limitation, free amino groups in SPARC proteins, such the epsilon-amino group of lysine, can be conjµgated with reagents such as carodiimides or heterobiofunctional agents. Alternatively, e.g., SPARC suflhydryl groups can be used for conjµgation. In addition, sµgar moieties bound to SPARC glycoproteins, can be oxidized to form aldehydes groups useful in a number of coupling procedures known in the art. The conjµgates formed in accordance with the invention can be stable in vivo or labile, such as enzymatically degradeable tetrapeptide linakages or acid-labile cis-aconityl or hydrazone linkages.

For use *in vivo,* the chemotherapeutic agent coupled to a compound or ligand capable of binding the SPARC protein desirably is formulated into a pharmaceutical composition comprising a physiologically acceptable carrier. Any suitable physiologically acceptable carrier can be used within the context of the invention, and such carriers are well known in the art.

The carrier typically will be liquid, but also can be solid, or a combination of liquid and solid components. The carrier desirably is a physiologically acceptable (e.g., a pharmaceutically or pharmacologically acceptable) carrier (e.g., excipient or diluent). Physiologically acceptable carriers are well known and are readily available. The choice of carrier will be determined, at least in part, by the location of the target tissue and/or cells, and the particular method used to administer the composition.

IV. Modes of Administering the Therapeutic and Targeting Embodiments

Typically, such compositions can be prepared as injectables, either as liquid solutions or suspensions; solid forms suitable for using to prepare solutions or suspensions upon the addition of a liquid prior to injection can also be prepared; and the preparations can also be emulsified. The pharmaceutical formulations suitable for injectable use include sterile aqueous solutions or dispersions; formulations containing known protein stabilizers and lyoprotectants, formulations including sesame oil, peanut oil or aqueous propylene glycol, and sterile powders for the extemporaneous preparation of sterile injectable solutions or dispersions. In all cases, the formulation must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxycellulose. Dispersions can also be prepared in glycerol, liquid polyethylene glycols, and mixtures thereof and in oils. Under ordinary conditions of storage and use, these preparations contain a preservative to prevent the growth of microorganisms.

The chemotherapeutic agent (e.g., SPARC therapy) coupled to a SPARC protein can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such as organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like.

The composition can further comprise any other suitable components, especially for enhancing the stability of the composition and/or its end-use. Accordingly, there is a wide variety of suitable formulations of the composition of the invention. The following formulations and methods are merely exemplary and are in no way limiting.

Formulations suitable for administration via inhalation include aerosol formulations. The aerosol formulations can be placed into pressurized acceptable propellants, such as dichlorodifluoromethane, propane, nitrogen, and the like. They also can be formulated as non-pressurized preparations, for delivery from a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of a sterile liquid excipient, for example, water, for injections, immediately prior to use. Extemporaneous injection solutions and suspensions can be prepared from sterile powders, granules, and tablets of the kind previously described.

Formulations suitable for anal administration can be prepared as suppositories by mixing the active ingredient with a variety of bases such as emulsifying bases or watersoluble bases. Formulations suitable for vaginal administration can be presented as pessaries, tampons, creams, gels, pastes, foams, or spray formulas containing, in addition to the active ingredient, such carriers as are known in the art to be appropriate.

In addition, the composition can comprise additional therapeutic or biologically-active agents. For example, therapeutic factors useful in the treatment of a particular indication can be present. Factors that control inflammation, such as ibuprofen or steroids, can be part of the composition to reduce swelling and inflammation associated with *in vivo* administration of the pharmaceutical composition and physiological distress.

The following examples further illustrate the invention but should not be construed as in any way limiting its scope.

### EXAMPLE 1

This example demonstrates the specific binding of anti-SPARC antibody to SPARC.

Whole cell extract was prepared from HUVEC cells by sonication. The protein was separated on a s5-15% SDS-PAGE, transferred onto PVDF membrane and visualized with a polyclonal antibody against SPARC and a monoclonal antibody against SPARC. Both antibodies reacted to a single band at 38 kDa, the correct molecular weight for SPARC. When MX-1 tumor cell line was analyzed by the same method, SPARC was detected in both the clarified cell lysate or the membrane rich membrane fraction.

### EXAMPLE 2

This example demonstrates the absence of SPARC expression in normal tissues.

Normal human and mouse tissue were immunostained and scored (0-4) for SPARC staining using a tumor and normal tissue array. Immunostaining was performed using polyclonal rabbit anti-SPARC antibody. SPARC was not expressed in any of the normal tissues, with the exception of the esophagus. Likewise, SPARC was not expressed in any of the normal mouse tissue, except the kidney of the female mouse. However, it is possible that this expression was due to follistatin which is identical to SPARC.

**Table 1. SPARC Expression in Human Normal Tissues**

| | |
|---|---|
| Stomach | 0/8 |
| Colon | 0/9 |
| Rectum | 0/15 |
| Liver | 0/14 |
| Spleen | 0/10 |
| Lung | 0/14 |
| Kidney | 1/14 |
| Brain | 1/14 |
| Testis | 0/8 |
| Prostate | 0/3 |
| Heart | 0/9 |
| Tonsil | 0/10 |
| Lymph Nodes | 0/10 |
| Appendix | 0/10 |
| Esophagus | 5/5 |
| Pancreas | 0/5 |
| Eyeball | 0/5 |
| Ovary | 0/5 |

**Table 2. SPARC Expression in Mouse Normal Tissues**

| | |
|---|---|
| Liver | 0/19 |
| Kidney (M) | 0/8 |
| Kidney (F) | 6/8 |
| Lung | 0/16 |
| Muscle | 0/20 |
| Brain | 0/20 |
| Heart | 0/18 |
| Stomach | 0/20 |
| Spleen | 0/20 |

### EXAMPLE 3

This example illustrates the expression of SPARC in MX-1 tumor cells.

MX-1 cells were cultured on a coverslip and stained with an antibody directed against human SPARC using methods known in the art. Antibody staining was observed, which demonstrates that MX-1 is expressing SPARC. These results sµggest that SPARC expression detected in MX-1 tumor cells is a result of SPARC secretion by MX-1 tumor cells. Staining was more intense for MX-1 tumor cells than that of normal primary cells such as HUVEC (human umbiblical vein endothelial cells), HLMVEC (Human lung microvessel endothelial cells), and HMEC (Human mammary epithelial cells). Thoµgh the majority of the SPARC staining was internal SPARC, significant level of surface SPARC was detected as demonstrated by confocal miscroscopy and staining of unpermeabilized cells.

### EXAMPLE 4

This example demonstrates SPARC binding to albumin throµgh the direct binding of fluorescent-tagged albumin to filter immobilized SPARC.

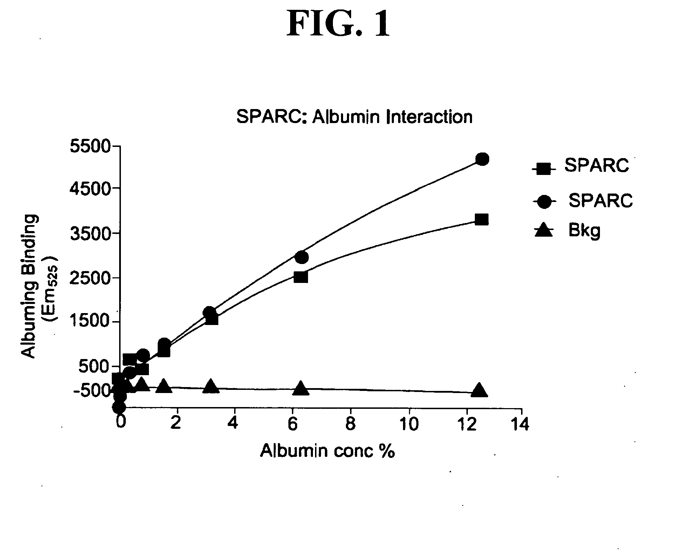

Purified SPARC was immobilized onto PVDF membrane and reacted with increasing concentration of Human Serum Albumin/Bovine Serum Albumin (HSA/BSA) which has been Alexa 488 flourochrome labeled. Binding was demonstrated with IC₅₀ at about the equivalent of a plasma concentration of HSA of 5% (weight/volumne) (FIG. 1).

Specifically the following protocol is used:
1) Incubate membrane with 30% methanol for 5 min, using Sterile MultiScreen (HTS) 96-well Filtration system from Millipore (Cat. No.: MSIPS4510);
2) Wash twice with Hanks Balance Salt Solution (HSBS);
3) Incubate with 100 µl of 5 µg/100 µl solution of purified SPARC in HSBS;
4) After 1 houtr at 25 °C, wash off twice with HSBS;
5) Block with 5% milk overnight (5% Non-fat dry milk (Carnation) in 1x TBS) at 4 °C.
6) Wash twice with HSBS;
7) Incubate with albumin (resuspend 5 mg of BSA-Alexa fluor 488 (Molecular Probes) with 1 ml of HSA injection 25%, BAXTER);
8) After 1 hrour, wash three times with HSBS;
9) Read with with a fluorometer using detection wavelength for Alexa fluor 488;
10) Specific binding is total binding minus binding to membrane without SPARC; and
11) Plot specific binding versus HSA concentration (%).

The results also demonstrate that SPARC accumulated in a tumor could serve as a sink for HSA.

### EXAMPLE 5

This example illustrates the co-localization of SPARC with albumin in an MX-1 tumor xenograft.

Paclitaxel albumin nanoparticles (Abraxane, ABX or ABI-007) have been shown to have an improved response rate over Taxol (TAX) in a Phase 3 metastatic breast cancer trial (33% vs. 19%, p<0.0001) (see, e.g., O'Shaµgbnessy, SABCS). Albumin-mediated transendothelial transport of paclitaxel (P) and increased intratumoral accumulation of paclitaxel for ABX versus TAX was demonstrated recently (see, e.g., Desai, SABCS 2003). Albumin binds to SPARC (see, e.g., Schnitzer, J. Biol. Chem., 269, 6072-82 (1994)).

The MX-1 tumor cell line is derived from a human breast cancer. Serial cryosections of human MX-1 tumor xenograft, human primary breast tumor tissues (n=141), and normal human breast tissue (n=115) were immunostained and scored (0-4) for albumin, SPARC (using anti-SPARC antibody), and caveolin-1 staining. Cultured MX-1 cells also were immunostained for SPARC. Paclitaxel albumin nanoparticles (Abraxane, ABX or ABI-007) and Taxol (TAX) were prepared with radioactive paclitaxel (P) (20 mg/kg IV), and were used to determine the biodistribution of paclitaxel in normal tissues of athymic mice.

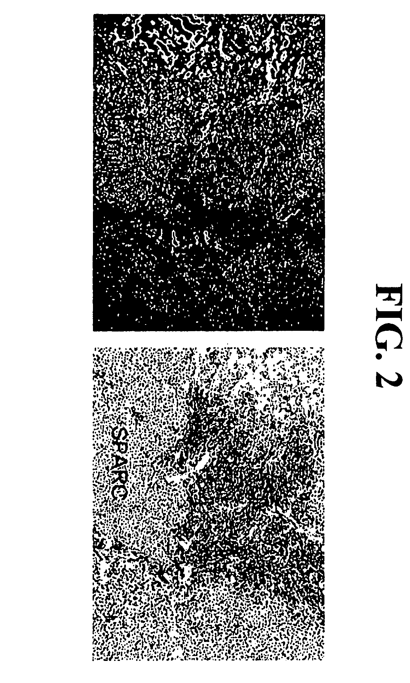

Albumin staining in the MX-1 tumor was focal and co-localized with SPARC (FIG. 2). Caveolin-1 staining confirmed that blood vessel density in albumin-containing areas was no different from albumin-free areas. SPARC expression by MX-1 cultured cells was confirmed by positive staining with anti-SPARC antibody. Paclitaxel accumulation in normal tissues (SPARC negative) was significantly lower for ABX as compared to TAX (p<0.004) for 7/10 tissues. 46% of the human primary breast tumors exhibited strong SPARC staining (score >2), as compared to 1% for normal tissues (p<0.0001). In a subset of 50 tumor tissues, SPARC expression did not correlate with staging, ER status, or PgR status; however, there was trend for high SPARC expression among p53-negative tumors.

The co-localization of albumin and SPARC sµggests that SPARC, by its albumin binding activity, may behave as an intratumoral target for albumin binding in breast tumors. As transport of paclitaxel in ABX is dependent on albumin (see, e.g., Desai SABCS, 2003), this may explain the improved tumor accumulation of ABX as compared to TAX. ABX accumulation in normal tissues was lower than for TAX, consistent with lack of SPARC expression in normal tissues. Screening of patients for SPARC allows for the identification of patients more responsive to ABX. The presence of SPARC in these tumors allows for targeting and therapy using anti-SPARC antibody.

### EXAMPLE 6

This example illustrates endothelial receptor (gp60)-mediated caveolar transcytosis of paclitaxel albumin nanoparticles (ABI-007).

Paclitaxel (P) albumin nanoparticles (Abraxane, ABX or ABI-007) demonstrated improved response rate over Taxol in a phase III metastatic breast cancer trial (33% vs 19%, p<0.0001) (SABCS, O'Shagghnessy et al, 2003). Cremophor in Taxol (TAX) entraps P in micelles in plasma, reducing the paclitaxel available for cellular partitioning (see, e.g., Sparreboom et al., Cancer. Res., 59, 1454 (1999)). Studies in athymic mice have shown 30-40% higher intratumor paclitaxel concentrations with ABX as compared to equal doses of TAX (SABCS, Desai et al, 2003). Albumin is transported across endothelial cells (EC) by specific receptor (gp60)-mediated caveolar transport (see, e.g., John et al., Am. J. Physiol., 284, L187 (2001)). It was hypothesized that albumin-bound paclitaxel in ABX may be transported across tumor microvessel EC by gp60, and this mechanism may be particularly active for ABX as compared to TAX.

A series of experiments were performed to evaluate binding and transport of paclitaxel by human umbilical vein endothelial cells (HUVEC) and human lung microvessel endothelial cells (HLMVEC) for ABX and TAX. Fluorescent paclitaxel (FP) was used as a probe and fluorescent ABX and TAX were formulated with FP to probe the binding and transport of paclitaxel across EC monolayers grown on a transwell apparatus.

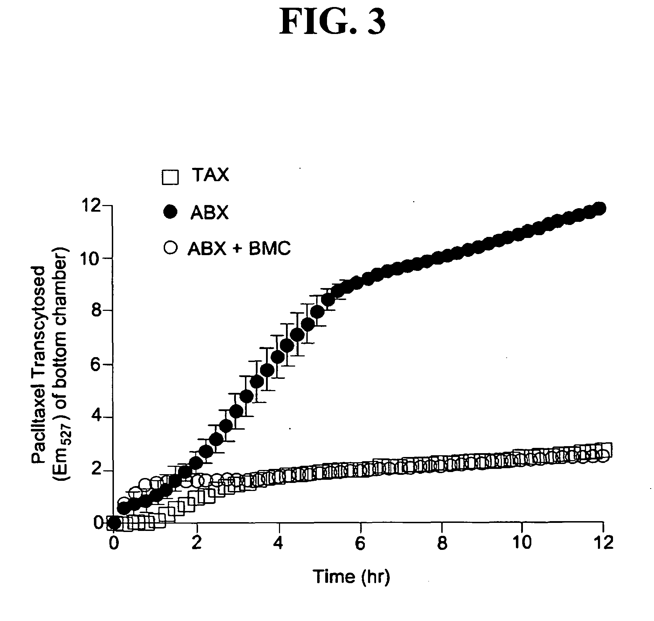

Binding of paclitaxel to cells (HUVEC) was 10X higher for ABX than TAX. The transport of paclitaxel from ABX across EC monolayers was enhanced by 2-3 fold and 2-4 fold for HUVEC and HMVEC, respectively, as compared to TAX. Transport was dependent on albumin. Transport of paclitaxel from ABX was inhibited by the presence of anti-SPARC antibody, which is known to bind gp60, the receptor required for caveolar albumin transcytosis. Known inhibitors of calveolar transcytosis, NEM and beta-methyl cyclodextrin (BMC), also inhibited the transport of paclitaxel from ABX across the endothelial monolayers (FIG. 3). Inhibition of caveolar transport decreased transport of P from ABX to the level of TAX transport.

These results demonstrate that paclitaxel from ABX is actively transported across EC by gp60-mediated caveolar transcytosis, whereas P from TAX appears to be transported at a 2-4 fold lower rate primarily by a paracellular (non-caveolar) mechanism. This pathway may in part be responsible for increased intratumoral concentrations of paclitaxel seen for ABX relative to TAX. Cremophor in TAX inhibits transcytosis of paclitaxel across endothelial cells.

### EXAMPLE 7

This example illustrates the internalization of labeled albumin into MX-1 tumor cells and colocalization within the MX-1 cell with intracellular SPARC expression.

MX-1 cells were cultured on a coverslip and permeabilized with suitable agents. Cells were exposed to fluorescent albumin and following washing were exposed to SPARC antibody. This was followed by exposure to a secondary antibodies having a different fluorescent tag than the albumin. It was surprisingly observed that the labeled albumin colocalised with the presence of SPARC within the cell indicating that albumin was rapidly internalized and targeted intracellular SPARC.

### EXAMPLE 8

This example demonstrates an increase in endothelial transcytosis via gp60 (albumin receptor) of pharmaceutical compositions comprising paclitaxel and albumin as compared to Taxol..

Human lung microvessel endothelial cells (HLMVEC) were grown to confluence on a transwell. The inventive pharmaceutical composition comprising paclitaxel and albumin, or Taxol containing fluorescent paclitaxel (Flutax) at a concentration of 20 µg/mL, was added to the upper transwell chamber.

The transport of paclitaxel by transcytosis from the upper chamber to the lower chamber was monitored continuously using a fluorometer. A control containing only Flutax without albumin was also used. The control with Flutax showed no transport, validating the integrity of the confluent HLMVEC monolayer. Transport of paclitaxel from the albumin-paclitaxel composition was much faster than paclitaxel from Taxol in the presence of 5% HSA (physiological concentration). Transport rate constants (Kₜ) for the albumin-paclitaxel composition and Taxol were 1.396 h⁻¹ and 0.03 h⁻¹, respectively. The total amount of paclitaxel transported across the monolayer was three times higher for the albumin-paclitaxel composition than Taxol. Thus, the use of albumin or other suitable mimetic including aantibodies or fragments against the gp60 recepetor or other endothelial cell receptor can assist in the transport of a desired therapeutic agent across the endothelial barrier into the tumor interstitium.

### EXAMPLE 9

This example illustrates the overexpression of SPARC protein in human breast carcinoma cells.

SPARC expression in human breast carcinoma cells was determined using a tumor array from Cybrdi, Inc. (Gaithersburg, MD). The results of this analysis are set forth in Table 1. Intensity of staining was scored from "Negative" to 4+, with the higher number corresponding to greater intensity of overexpression. 49% of breast carcinoma stained positive (2+ and above) for SPARC, as compared to 1% of normal tissue (p<0.0001).

**Table 3. SPARC Expression in Breast Cancer**

| | **SPARC Staining (%)** | | | | | |
|---|---|---|---|---|---|---|
| | Negative | -/+ | 1+ | 2+ | 3+ | 4+ |
| Carcinoma Cells | 31 (34%) | 14 (15%) | 1 (1%) | 11 (12%) | 9 (10%) | 25 (27%) |
| Normal Cells | 93 (89%) | 7 (7%) | 4 (4%) | 1 (1%) | 0 (0%) | 0 (0%) |

### EXAMPLE 10

This example demonstrates SPARC overexpression in squamous cell head and neck cancers with high response rates using nanoparticle albumin-bound paclitaxel (ABI-007).

In phase I and II clinical studies of patients with squamous cell carcinoma (SCC) of head and neck (H&N) and anal canal, response rates of 78% and 64% were observed, respectively, for intra-arterially delivered Nanoparticle Albumin-Bound Paclitaxel (Abraxane^{®}, ABX or ABI-007) (see, e.g., Damascelli et al., Cancer, 92(10), 2592-2602 (2001), and Damascelli et al., AJR, 181, 253-260 (2003)). In comparing in vitro cytoxicity of ABX and Taxol (TAX), we observed that a squamous cervix (A431) line demonstrated improved IC₅₀s for ABX (0.004 µg/ml) vs TAX (0.012 µg/ml). Albumin-mediated transendothelial caveolar transport of paclitaxel (P) and increased intratumoral accumulation of P for ABX versus TAX was demonstrated recently (see, e.g., Desai, SABCS 2003).

Human H&N tumor tissues (n=119) and normal human H&N tissue (n=15) were immunostained and scored (0-4+) for SPARC staining using a tumor and normal tissue array. Immunostaining was performed using polyclonal rabbit anti-SPARC antibody. In a new phase I dose escalation study (ABX given IV over 30 minutes q3w), a subset of head and neck cancer patients (n=3) were analyzed for response to ABX.

SPARC was overexpressed (score > 2+) in 60% (72/119) of the H&N tumors versus 0% (0/15) in normal tissues (p<0.0001). In the phase I study, 2/3 H&N patients achieved partial response (PR) after 2 cycles of treatment at dose levels of 135 mg/m² (1 pt) and 225 mg/m² (1 pt). A third patient at 260 mg/m² progressed.

SPARC was found to be overexpressed in 60% of squamous cell H&N tumors. This may explain the high single-agent activity of ABX seen previously in squamous cell H&N cancers due to binding of albumin-bound paclitaxel to SPARC expressed in these tumors. 2/3 patients with squamous cell H&N tumors achieved PR in a new phase I study. Human H&N tumor tissues (n=119) and normal human H&N tissue (n=15) were immunostained and scored (0-4+) for SPARC staining using a tumor and normal tissue array. Immunostaining was performed using polyclonal rabbit anti-SPARC antibody. SPARC was overexpressed (score > 2+) in 60% (72/119) of the H&N tumors versus 0% (0/15) in normal tissues (p<0.0001). This may explain the high single-agent activity of ABX seen previously in squamous H&N cancers due to binding of albumin-bound paclitaxel to SPARC expressed in these tumors.

In a new phase I dose escalation study (ABX given IV over 30 minutes *q3w*), a subset of head and neck cancer patients (n=3) were analyzed for response to ABX. In the phase I study, 2/3 H&N patients achieved partial response (PR) after 2 cycles of treatment at dose levels of 135 mg/m² (1 pt) and 225 mg/m² (1 pt). A third patient at 260 mg/m² progressed. Tumor tissues from these patients were stained for SPARC and 1 of the responding patients showed strong overexpression for SPARC.

### EXAMPLE 11

This example demonstrates correlation of SPARC overexpression with high response rates using nanoparticle albumin-bound paclitaxel (ABI-007) in squamous head and neck cancers.

In another phase II clinical study of 54 patients with squamous cell carcinoma of head and neck treated with intra-arterial ABX, an overall response rate of 78% was noted. Cancer biopsies from 16 patients in this study receiving intra-arterial ABX were evaluated for SPARC expression and correlation with clinical response. Staining with anti-SPARC polyclonal antibody (R&D Systems, Minneapolis, MN, USA) was scored on a 0-4 scale (0= no staining, 4+= strong positive). Positive SPARC expression was identified as > 2+ staining and negative SPARC expression was identified as < 2+ staining. The ABX - responders exhibited higher incidence of SPARC expression (10/11, 91%) versus nonresponders (2/5, 40%) (p=0.06.) ABX -response was significantly higher for SPARC-positive patients (10/12 = 83%) versus SPARC-negative patients (1/4 = 25%) (p=0.06). In addition, the SPARC-negative patients exhibited significantly lower response rate than the overall response rate in the study (including patients treated with ABX or other chemotherapeutic agents) (1/4, 25% vs. 42/54, 78%; p < 0.05)).

### EXAMPLE 12

This example demonstrates that tumor cells expressing SPARC are more sensitive to Abraxane^{®} than tumor cells which do not express SPARC.

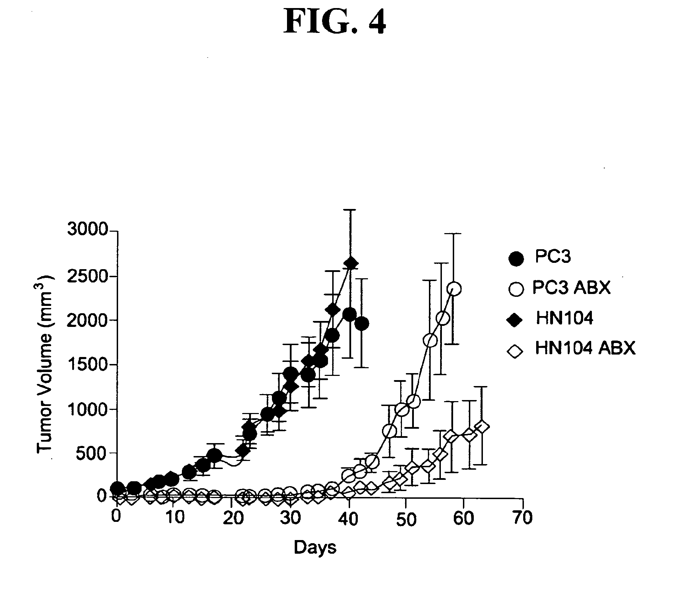

An expression vector with CMV promoter driving the expression of SPARC was transfected into PC3 cells. Stable integrants with high SPARC expression were selected by G418 (at 500 µg/ml of culture media). One of these clone, HN104, exhibited high SPARC expression by RT-PCR and by Western blot. This clone was grown in athymic nude mice as xenograft. The growth and response of HN104 to Abraxane^{®} ("ABX" in FIGS. 4) were compared to the growth and response of the parent cell line PC3 to Abraxane^{®}. Abraxane^{®} was dosed when tumor reached 100 mm³ at dose level of 15 mg/kg per day for five days.

The HN104 exhibited a longer lag phase versus the parent PC3 xenograft. Growth was similar upon completion of the lag phase, as the tumor curves of PC3 and HN104 were similar when the HN104 was shifted to the left by 2 weeks (FIG. 4). As shown in FIG. 4 (FIG. 4 depicts tumor volume adjusted for the SPARC-induced lag phases in the transfected cells by shifting the HN104 curves 20 days to the left), the cell line overexpressing SPARC exhibited significantly greater sensitivity to Abraxane^{®} than the parent cell line. For tumor volumes 100-800 mm³; the average day separating equivalent sized tumor volumes in the treated versus untreated was 25 days for PC3 and 36 days for HN104. Thus, SPARC sensitizes prostatic cancer cells to Abraxane^{®}.

### EXAMPLE 13

To further explore the chemotherapy sensitizing activity of SPARC, the athymic mouse tumor xenograft system was used to assess the sensitization of HT 29 human colocancer cells to 5-flurouracil ("5-FU").

The cancer cells were implanted subcutaneously bilaterally in athymic mice. The mice were treated with saline or 25 mg/kg of 5-FU (see Table 4). Those animals that received SPARC were treated with wild type polypeptide (SEQ ID NO:1) at a dose of 4 mg/kg, 6 mg/kg or 8 mg/kg (see Table 4). The animals were monitored for survival and xenograft growth as measures of efficacy and body weight change as a measure of toxicity. The results are summarized in the following Table:

**Table 4**

| **Anti-tumor activity of 5-FU alone and in combination with BIO1 in the s.c. human HT29 colon cancer xenograft model in nude mice** | | | |
|---|---|---|---|
| **Agent** | **Dose (mg/kg)^{a}** | **TGI (%)^{b,c}** | **% BWLmax** |
| **Saline** | **0** | **-** | **-1.3** |
| **5-FU** | **25** | **79.8** | **-5.8** |
| | | | |
| **5-FU + BI01 (200 µg)** | **25 + 8** | **10.4** | **-3.3** |
| **5-FU + BI01 (150 µg)** | **25 + 6** | **47.4** | **-7.4** |
| **5-FU + BI01 (100 µg)** | **25 + 4** | **50.8** | **-6.5** |

| | | | |
|---|---|---|---|
| ^{a}5-FU, IP: Q2Dx3/2 weeks (2 cycles); BIO1, IP; Q3Dx2/6 weeks ^{b}TGI, tumor growth inhibition ^{c}Post-treatment day 25. | | | |

[0102] FIG. 5 shows the tumor volume curves for the group of animals treated with wild type SPARC ("BIO1 ") and their controls. Wild type SPARC failed to sensitize the cancer cells to 5-FU at any of the SPARC concentrations used.

[0103] This example demonstrates that SPARC is not a universal sensitizer and that its sensitizing activity may be dependant on the biology of the cancer cells and/or the therapy administered.

### EXAMPLE 14

An *in vitro* angiogenesis assay (TSC CellWorks Angiokit) was used to assess SPARC's angiogenic activity, which surprisingly showed that SPARC's angiogenic activity is concentration dependent.

In this system, human umbilical vein endothelial cells ("HUVEC") are co-cultured with other human cells (fibroblast) in collagen matrix. HUVEC cells initially form small islands within the culture matrix. HUVEC differentiate, proliferate and enter migratory phase during which they move through the matrix and form threadlike tubule structures (9-11 days). These gradually join up and form a network which resemble the capillary bed.

HUVEC cells grown in a three dimensional matrix were treated with various concentration of wild type SPARC polypeptide (SEQ ID NO: 1) or the Q3 mutant SPARC polypeptide (SEQ ID NO: 3). Capillary microtubules were visualized using immunostaining for CD31 after 12 days in culture. FIG. 6 depicts SPARC's concentration dependent effect on neoangiogensis showing characteristic examples of the capillary microtubular density in the absence of SPARC (0 µg/ml), wild type or Q3 SPARC at 10 µg/ml, and wild type or Q3 SPARC at 100 µg/ml.

Surprisingly, SPARC at 10 µg/ml is proangiogenic, while SPARC at 100 µg/ml inhibits angiogenesis.

### EXAMPLE 15

This example demonstrates a synergistic effect of combining SPARC, a putative angiogenesis inhibitor and Abraxane^{®} in a tumor xenograft model.

In view of the surprising finding presented in EXAMPLE 14 that SPARC's angiogenic activity is concentration dependent. Applicants postulated that at lower concentration SPARC's anti-cancer activities may be masked by its strong proangiogenic activity. To test this, the effect of adding an angiogenesis inhibitor to a regimen of SPARC plus Abraxane^{®} was studied.

Human breast cancer (FIGS. 7 and 8) and colon cancer cells (FIGS. 9 and 10) were grown in athymic nude mice as xenografts as in EXAMPLES 12 and 13. There were 5 mice in each treatment group. Tumors were treated when they reached 200 mm³. The mice were treated with either: saline, as a negative control; SPARC (150 µg/kg 2x/week), alone; the angiogenesis inhibitor Avastin (4mg/kg twice per week), alone; Abraxane^{®} (15 mg/kg every fourth day for three treatments), alone; SPA RC and Abraxane^{®} or SPARC, Avastin and Abraxane^{®}. Both wild type ("BI01") and Q3 mutant SPARC ("BIO2") were evaluated. The animals were monitored for survival and xenograft growth as measures of efficacy and body weight change as a measure of toxicity. The results are summarized in the following Table showing the percent inhibition of tumor growth beyond that caused by Abraxane^{®} alone:

**Table 5. Tumor growth inhibition (TGI) relative to Abraxane alone .**

| | Avastin | BIO1 + Abraxane | BIO2 + Abraxane | BIO1 + Abraxane + Avastin | BIO2 + Abraxane + Avastin |
|---|---|---|---|---|---|
| Breast Cancer (MDA-MB-231) | 16% | 82% | 69% | 92% | 86% |
| Colon Cancer (HT29) | <0% | 39% | 9% | 76% | 33% |

For wild type SPARC and breast cancer cells single agent therapy was no better than the saline control treatments. As FIG. 8 shows the addition of SPARC or of SPARC and Avastin produced marked decreases in tumor growth as compared to the other arms of the study. FIG. 9 shows that the Q3 mutant SPARC gave similar, though blunted, results.

For wild type SPARC and colon cancer cells single agent therapy with SPARC or Avastin was no better than the saline control treatments. Single agent treatment with Abraxane^{®} resulted in a significant improvement in tumor growth over control. As FIG. 10 also shows the addition of SPARC or of SPARC and Avastin produced further decreases in tumor growth as compared to Abnaxane^{®} alone. However, FIG. 10 shows that the Q3 mutant SPARC fails to slow tumor growth beyond that achieved with Abraxane^{®} and the addition of Avastin and Q3 SPARC results in only a mild improvement in controlling tumor growth. Based on changes in body weight, no regimen was significantly more toxic than any other regimen.

In another system, Sutent, another angiogenesis inhibitor produced the same results. Again, 5 athymic mice each were subcutaneously injected with human breast cancer cells. Treatment started when the tumors grew to 100 mm³. Therapy consisted of either Sutent or Sutent with wild type SPARC ("BIOI "), the results are shown in Table 5 (showing the percent suppression of tumor growth below that cause by Abraxane^{®} alone) and FIG. 11.

**Table 6. Tumor growth inhibition (TGI) relative to Abraxane alone.**

| | Abraxane | Abraxane |
|---|---|---|
| | +Sutent | +Sutent |
| | | +BIO1 |
| MDA-MB-231 | 0% | 76% |

As with Avastin, using Sutent as an angiogenesis inhibitor, SPARC and Abraxane^{®} combination appears to have a synergistic effect.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

## Claims

1. A composition comprising a SPARC polypeptide, an angiogenesis inhibitor and albumin-bound paclitaxel for use in treating a mammalian tumor.

2. The composition for use according to claim 1, wherein the SPARC polypeptide is a polypeptide comprising the amino acid sequence of SEQ ID NOS. 1, 3 or a combination thereof.

3. The composition for use according to claim 2, wherein the SPARC polypeptide is present at a concentration of from 10 µg/ml to 100 mg/ml.

4. The composition for use according to claim 1, wherein more than 50% of the albumin-bound paclitaxel is in nanoparticle form.

5. The composition for use according to claim 1, wherein the albumin-bound paclitaxel is present at a concentration of from 10 mg/ml to 100 mg/ml, or at a concentration of from 1 mg/ml to 10 mg/ml, or at a concentration of from 0.1 mg/ml to 1 mg/ml.

6. The composition for use according to claim 1, wherein the angiogenesis inhibitor is an inhibitor of mTOR, Aurora kinase, an inhibitor of VEGFR kinase, an inhibitor of PDGFR kinase, sorafenib, Sutent, Axitinib, Avastin, marimastat, bevacizumab, carboxyamidotriazole, TNP- 470, CM101, IFN-α, IL-12, platelet factor-4, suramin, SU5416, thrombospondin, VEGFR antagonists, angiostatic steroids, cartilage-derived angiogenesis inhibitory factor, matrix metalloproteinase inhibitors, angiostatin, endostatin, 2-methoxyestradiol, tecogalan, thrombospondin, prolactin, αvβ3 inhibitors, tecogalan, BAY 12-9566, AG3340, CGS27023A, COL-3, vitaxin, ZD0101, TNP-40, thalidomide, squalamine, IM862, PTK787, fumagillin, analogues of fumagillin, BB-94, BB-2516, linomid, antibodies to vascular growth factors, antibodies to vascular growth factor receptors or a combination thereof, preferably the angiogenesis inhibitor is Avastin.

7. The composition for use according to claim 6, wherein the angiogenesis inhibitor is Avastin at a concentration of from 10 mg/ml to 50 mg/ml.

8. The composition for use according to claim 1, wherein the SPARC polypeptide is a polypeptide comprising the amino acid sequence of SEQ ID NO. 1.

9. The composition for use according to claim 1, wherein the SPARC polypeptide is for administration at a dose of from 40 µg/kg to 40 mg/kg with a dosing cycle of at least 1 week.

10. The composition for use according to claim 1, wherein the SPARC polypeptide is for administration within 12 hours of the administration of the albumin-bound paclitaxel, or more than 12 hours from the administration of the albumin-bound paclitaxel.

11. The composition for use according to claim 1, wherein the SPARC polypeptide and the albumin-bound paclitaxel are for substantially simultaneous administration.

12. The composition for use according to claim 1, wherein the SPARC polypeptide and the albumin-bound paclitaxel are included in a single dosage form.

13. The composition for use according to claim 1, wherein the SPARC polypeptide and the albumin-bound paclitaxel are for intravenous administration.

14. The composition for use according to claim 1, wherein the dose of albumin-bound paclitaxel is from 30 mg/m² to 1000 mg/m² with a dosing cycle of at least 1 week, or from 1 mg/m² to 30 mg/m² with a dosing cycle of at least 1 week, or from 0.3 mg/m² to 1 mg/m² with a dosing cycle of at least 1 week, or from 0.1 mg/m² to 0.3 mg/m² with a dosing cycle of at least 1 week.

15. The composition for use according to claim 1, wherein the composition reduces the tumor growth rate by 50 percent.

16. The composition for use according to claim 1, wherein the mammalian tumor is selected from the group consisting of oral cavity tumors, pharyngeal tumors, digestive system tumors, the respiratory system tumors, bone tumors, cartilaginous tumors, bone metastases, sarcomas, skin tumors, melanoma, breast tumors, the genital system tumors, urinary tract tumors, orbital tumors, brain and central nervous system tumors, gliomas, endocrine system tumors, thyroid tumors, esophageal tumors, gastric tumors, small intestinal tumors, colonic tumors, rectal tumors, anal tumors, liver tumors, gall bladder tumors, pancreatic tumors, laryngeal tumors, tumors of the lung, bronchial tumors, non-small cell lung carcinoma, small cell lung carcinoma, uterine cervical tumors, uterine corpus tumors, ovarian tumors, vulvar tumors, vaginal tumors, prostate tumors, prostatic carcinoma, testicular tumors, tumors of the penis, urinary bladder tumors, tumors of the kidney, tumors of the renal pelvis, tumors of the ureter, head and neck tumors, parathyroid cancer, Hodgkin's disease, Non-Hodgkin's lymphoma, multiple myeloma, leukemia, acute lymphocytic leukemia, chronic lymphocytic leukemia, acute myeloid leukemia, chronic myeloid leukemia and anal tumors.

17. The composition for use according to claim 8, wherein the SPARC polypeptide is for administration at a dose of from 0.1 to 100 mg/kg per dose with a dosing cycle of at least 1 week.

18. The composition for use according to claim 6, wherein the angiogenesis inhibitor is Sutent, Avastin or a combination thereof, preferably the angiogenesis inhibitor is Avastin.

19. The composition for use according to claim 18, wherein the angiogenesis inhibitor is Avastin and is for administration at a dose of from 15 µg/kg to 15 mg/kg with a dosing cycle of at least 1 week.

20. The composition for use according to claim 6, wherein the angiogenesis inhibitor is Sutent and is for oral administration.

21. The composition for use according to claim 1, wherein the angiogenesis inhibitor is for administration within 12 hours of the administration of the albumin-bound paclitaxel, or more than 12 hours from the administration of the albumin-bound paclitaxel.

22. The composition for use according to claim 1, wherein the angiogenesis inhibitor and albumin-bound paclitaxel such as taxane are for substantially simultaneous administration.

23. The composition for use according to claim 1, wherein the SPARC polypeptide, angiogenesis inhibitor and albumin-bound paclitaxel are included in a single dosage form.

24. The composition for use according to claim 1, wherein the SPARC polypeptide, the angiogenesis inhibitor and the albumin-bound paclitaxel are for intravenous administration.

25. The composition for use according to claim 1, wherein the combination reduces the tumor growth rate by at least 50% compared to albumin-bound paclitaxel monotherapy.

26. The composition for use according to claim 1, wherein the SPARC polypeptide is at a dose of 40 µg/kg to 40 mg/kg per dose, the angiogenesis inhibitor is Avastin at a dose of from 15 µg/kg to 15 mg/kg, and the albumin-bound paclitaxel is at a dose of
(a) from 30 mg/m² to 1000 mg/m² with a dosing cycle of at least 1 week,
(b) from 1 mg/m² to 30 mg/m² with a dosing cycle of at least 1 week,
(c) from 0.3 mg/m² to 1 mg/m² with a dosing cycle of at least 1 week, or
(d) from 0.1 mg/m² to 0.3 mg/m² with a dosing cycle of at least 1 week.

27. The composition for use according to claim 26, wherein the SPARC polypeptide comprises SEQ ID NO: 1.

28. The composition for use according to claim 26, wherein the SPARC polypeptide comprises SEQ ID NO: 3.

29. The composition for use according to claim 1, wherein SPARC polypeptide is lyophilized.

30. The composition for use according to claim 1, wherein the albumin-bound paclitaxel is lyophilized.

31. The composition for use according to claim 6, wherein the angiogenesis inhibitor is Avastin and wherein the Avastin is lyophilized.

## Patentansprüche

1. Zusammensetzung, welche ein SPARC-Polypeptid, einen Angiogenese-Inhibitor und an Albumin gebundenes Paclitaxel umfasst, zur Verwendung beim Behandeln eines Tumors bei einem Säuger.

2. Zusammensetzung zur Verwendung gemäß Anspruch 1, wobei das SPARC-Polypeptid ein Polypeptid ist, welches die Aminosäuresequenz von SEQ ID NO. 1, 3 oder einer Kombination davon umfasst.

3. Zusammensetzung zur Verwendung gemäß Anspruch 2, wobei das SPARC-Polypeptid in einer Konzentration von 10 µg/ml bis 100 mg/ml vorliegt.

4. Zusammensetzung zur Verwendung gemäß Anspruch 1, wobei mehr als 50 % des an Albumin gebundenen Paclitaxels in Form von Nanopartikeln vorliegen.

5. Zusammensetzung zur Verwendung gemäß Anspruch 1, wobei das an Albumin gebundene Paclitaxel in einer Konzentration von 10 mg/ml bis 100 mg/ml oder in einer Konzentration von 1 mg/ml bis 10 mg/ml oder in einer Konzentration von 0,1 mg/ml bis 1 mg/ml vorliegt.

6. Zusammensetzung zur Verwendung gemäß Anspruch 1, wobei es sich bei dem Angiogenese-Inhibitor um einen Inhibitor von mTOR, Aurora-Kinase, einen Inhibitor der VEGFR-Kinase, einen Inhibitor der PDGFR-Kinase, Sorafenib, Sutent, Axitinib, Avastin, Marimastat, Bevacizumab, Carboxyamidotriazol, TNP-470, CM101, IFN-α, IL-12, Plättchenfaktor 4, Suramin, SU5416, Thrombospondin, VEGFR-Antagonisten, angiostatische Steroide, von Knorpel abgeleiteten, die Angiogenese hemmenden Faktor, Matrix-Metalloproteinase-Inhibitoren, Angiostatin, Endostatin, 2-Methoxyestradiol, Tecogalan, Thrombospondin, Prolaktin, αvβ3-Inhibitoren, Tecogalan, BAY 12-9566, AG3340, CGS27023A, COL-3, Vitaxin, ZD0101, TNP-40, Thalidomid, Squalamin, IM862, PTK787, Fumagillin, Analoga von Fumagillin, BB-94, BB-2516, Linomid, Antikörper gegen Gefäß-Wachstumsfaktoren, Antikörper gegen Rezeptoren für Gefäß-Wachstumsfaktoren oder um eine Kombination davon handelt; vorzugsweise ist der Angiogenese-Inhibitor Avastin.

7. Zusammensetzung zur Verwendung gemäß Anspruch 6, wobei der Angiogenese-Inhibitor Avastin in einer Konzentration von 10 mg/ml bis 50 mg/ml ist.

8. Zusammensetzung zur Verwendung gemäß Anspruch 1, wobei das SPARC-Polypeptid ein Polypeptid ist, welches die Aminosäuresequenz von SEQ ID NO. 1 umfasst.

9. Zusammensetzung zur Verwendung gemäß Anspruch 1, wobei das SPARC-Polypeptid zur Verabreichung in einer Dosis von 40 µg/kg bis 40 mg/kg mit einer Dosierungsperiode von mindestens 1 Woche vorgesehen ist.

10. Zusammensetzung zur Verwendung gemäß Anspruch 1, wobei das SPARC-Polypeptid zur Verabreichung innerhalb von 12 Stunden ab der Verabreichung des an Albumin gebundenen Paclitaxels oder von mehr als 12 Stunden ab der Verabreichung des an Albumin gebundenen Paclitaxels vorgesehen ist.

11. Zusammensetzung zur Verwendung gemäß Anspruch 1, wobei das SPARC-Polypeptid und das an Albumin gebundene Paclitaxel zur im Wesentlichen simultanen Verabreichung vorgesehen sind.

12. Zusammensetzung zur Verwendung gemäß Anspruch 1, wobei das SPARC-Polypeptid und das an Albumin gebundene Paclitaxel in einer Einzeldosierungsform enthalten sind.

13. Zusammensetzung zur Verwendung gemäß Anspruch 1, wobei das SPARC-Polypeptid und das an Albumin gebundene Paclitaxel zur intravenösen Verabreichung vorgesehen sind.

14. Zusammensetzung zur Verwendung gemäß Anspruch 1, wobei die Dosis des an Albumin gebundenen Paclitaxels von 30 mg/m² bis 1000 mg/m² mit einer Dosierungsperiode von mindestens 1 Woche oder von 1 mg/m² bis 30 mg/m² mit einer Dosierungsperiode von mindestens 1 Woche oder von 0,3 mg/m² bis 1 mg/m² mit einer Dosierungsperiode von mindestens 1 Woche oder von 0,1 mg/m² bis 0,3 mg/m² mit einer Dosierungsperiode von mindestens 1 Woche reicht.

15. Zusammensetzung zur Verwendung gemäß Anspruch 1, wobei die Zusammensetzung die Wachstumsrate des Tumors um 50 % verringert.

16. Zusammensetzung zur Verwendung gemäß Anspruch 1, wobei der Tumor bei einem Säuger ausgewählt ist aus der Gruppe bestehend aus Tumoren der Mundhöhle, pharyngealen Tumoren, Tumoren des Verdauungssystems, Tumoren des respiratorischen Systems, Knochentumoren, Knorpeltumoren, Knochenmetastasen, Sarkomen, Hauttumoren, Melanom, Brusttumoren, Tumoren des Genitalsystems, Tumoren der Harnwege, Tumoren der Orbita, Tumoren des Gehirns und des Zentralnervensystems, Gliomen, Tumoren des endokrinen Systems, Schilddrüsentumoren, Ösophagustumoren, Magentumoren, Dünndarmtumoren, Dickdarmtumoren, Rektaltumoren, Analtumoren, Lebertumoren, Gallenblasentumoren, Pankreastumoren, Larynxtumoren, Tumoren der Lunge, Bronchialtumoren, nicht kleinzelligem Lungenkarzinom, kleinzelligem Lungenkarzinom, Tumoren der Cervix uteri, Tumoren des Corpus uteri, Ovarialtumoren, Vulvatumoren, Vaginaltumoren, Prostatatumoren, Prostatakarzinom, Hodentumoren, Penistumoren, Harnblasentumoren, Nierentumoren, Nierenbeckentumoren, Harnleitertumoren, Kopf- und Halstumoren, Nebenschilddrüsenkrebs, Morbus Hodgkin, Nicht-Hodgkin-Lymphom, multiplem Myelom, Leukämie, akuter lymphatischer Leukämie, chronischer lymphatischer Leukämie, akuter myeloischer Leukämie, chronischer myeloischer Leukämie und Analtumoren.

17. Zusammensetzung zur Verwendung gemäß Anspruch 8, wobei das SPARC-Polypeptid zur Verabreichung in einer Dosis von 0,1 bis 100 mg/kg pro Dosis mit einer Dosierungsperiode von mindestens 1 Woche vorgesehen ist.

18. Zusammensetzung zur Verwendung gemäß Anspruch 6, wobei es sich bei dem Angiogenese-Inhibitor um Sutent, Avastin oder eine Kombination davon handelt; vorzugsweise ist der Angiogenese-Inhibitor Avastin.

19. Zusammensetzung zur Verwendung gemäß Anspruch 18, wobei der Angiogenese-Inhibitor Avastin und zur Verabreichung in einer Dosis von 15 µg/kg bis 15 mg/kg mit einer Dosierungsperiode von mindestens 1 Woche vorgesehen ist.

20. Zusammensetzung zur Verwendung gemäß Anspruch 6, wobei der Angiogenese-Inhibitor Sutent und zur oralen Verabreichung vorgesehen ist.

21. Zusammensetzung zur Verwendung gemäß Anspruch 1, wobei der Angiogenese-Inhibitor zur Verabreichung innerhalb von 12 Stunden ab der Verabreichung des an Albumin gebundenen Paclitaxels oder von mehr als 12 Stunden ab der Verabreichung des an Albumin gebundenen Paclitaxels vorgesehen ist.

22. Zusammensetzung zur Verwendung gemäß Anspruch 1, wobei der Angiogenese-Inhibitor und das an Albumin gebundene Paclitaxel wie zum Beispiel Taxan zur im Wesentlichen simultanen Verabreichung vorgesehen sind.

23. Zusammensetzung zur Verwendung gemäß Anspruch 1, wobei das SPARC-Polypeptid, der Angiogenese-Inhibitor und das an Albumin gebundene Paclitaxel in einer Einzeldosierungsform enthalten sind.

24. Zusammensetzung zur Verwendung gemäß Anspruch 1, wobei das SPARC-Polypeptid, der Angiogenese-Inhibitor und das an Albumin gebundene Paclitaxel zur intravenösen Verabreichung vorgesehen sind.

25. Zusammensetzung zur Verwendung gemäß Anspruch 1, wobei die Kombination die Wachstumsrate des Tumors um mindestens 50 % im Vergleich mit einer Monotherapie mit an Albumin gebundenem Paclitaxel verringert.

26. Zusammensetzung zur Verwendung gemäß Anspruch 1, wobei das SPARC-Polypeptid in einer Dosis von 40 µg/kg bis 40 mg/kg pro Dosis vorliegt, der Angiogenese-Inhibitor Avastin in einer Dosis von 15 µg/kg bis 15 mg/kg ist und das an Albumin gebundene Paclitaxel in einer Dosis
(a) von 30 mg/m² bis 1000 mg/m² mit einer Dosierungsperiode von mindestens 1 Woche,
(b) von 1 mg/m² bis 30 mg/m² mit einer Dosierungsperiode von mindestens 1 Woche,
(c) von 0,3 mg/m² bis 1 mg/m² mit einer Dosierungsperiode von mindestens 1 Woche oder
(d) von 0,1 mg/m² bis 0,3 mg/m² mit einer Dosierungsperiode von mindestens 1 Woche
vorliegt.

27. Zusammensetzung zur Verwendung gemäß Anspruch 26, wobei das SPARC-Polypeptid SEQ ID NO. 1 umfasst.

28. Zusammensetzung zur Verwendung gemäß Anspruch 26, wobei das SPARC-Polypeptid SEQ ID NO. 3 umfasst.

29. Zusammensetzung zur Verwendung gemäß Anspruch 1, wobei das SPARC-Polypeptid lyophilisiert ist.

30. Zusammensetzung zur Verwendung gemäß Anspruch 1, wobei das an Albumin gebundene Paclitaxel lyophilisiert ist.

31. Zusammensetzung zur Verwendung gemäß Anspruch 6, wobei es sich bei dem Angiogenese-Inhibitor um Avastin handelt und wobei das Avastin lyophilisiert ist.

## Revendications

1. Composition comprenant un polypeptide SPARC, un inhibiteur de l'angiogenèse et du paclitaxel lié à de l'albumine, pour utilisation dans le traitement d'une tumeur de mammifère.

2. Composition pour utilisation selon la revendication 1, dans laquelle le polypeptide SPARC est un polypeptide comprenant la séquence d'acides aminés de la SEQ ID NO : 1 ou 3 ou une de leurs combinaisons.

3. Composition pour utilisation selon la revendication 2, dans laquelle le polypeptide SPARC est présent à une concentration de 10 µg/ml à 100 mg/ml.

4. Composition pour utilisation selon la revendication 1, dans laquelle plus de 50 % du paclitaxel lié à de l'albumine sont sous forme de nanoparticules.

5. Composition pour utilisation selon la revendication 1, dans laquelle le paclitaxel lié à de l'albumine est présent à une concentration de 10 mg/ml à 100 mg/ml, ou à une concentration de 1 mg/ml à 10 mg/ml, ou à une concentration de 0,1 mg/ml à 1 mg/ml.

6. Composition pour utilisation selon la revendication 1, dans laquelle l'inhibiteur de l'angiogenèse est un inhibiteur de mTOR, Aurora kinase, un inhibiteur de VEGFR kinase, un inhibiteur de PDGFR kinase, le sorafénib, le sutent, l'axitinib, l'avastin, le marimastat, le bévacizumab, le carboxyamidotriazole, TNP-470, CM101, IFN-α, IL-12,- le facteur plaquettaire 4, la suramine, SU5416, la thrombospondine, les antagonistes de VEGFR, les stéroïdes angiostatiques, le facteur inhibant l'angiogenèse dérivé du cartilage, les inhibiteurs de métalloprotéinases de matrice, l'angiostatine, l'endostatine, le 2-méthoxy-oestradiol, le técogalan, la thrombospondine, la prolactine, les inhibiteurs d'αvβ3, le técogalan, BAY 12-9566, AG3340, CGS27023A, COL-3, la vitaxine, ZD0101, TNP-40, le thalidomide, la squalamine, IM862, PTK787, la fumagilline, les analogues de fumagilline, BB-94, BB-2516, le linomide, les anticorps dirigés contre les facteurs de croissance vasculaires, les anticorps dirigés contre les récepteurs de facteurs de croissance vasculaires, ou une de leurs combinaisons, et de préférence l'inhibiteur de l'angiogenèse est l'avastin.

7. Composition pour utilisation selon la revendication 6, dans laquelle l'inhibiteur de l'angiogenèse est l'avastin à une concentration de 10 mg/ml à 50 mg/ml.

8. Composition pour utilisation selon la revendication 1, dans laquelle le polypeptide SPARC est un polypeptide comprenant la séquence d'acides aminés de la SEQ ID NO : 1.

9. Composition pour utilisation selon la revendication 1, dans laquelle le polypeptide SPARC est destiné à être administré à une dose de 40 µg/kg à 40 mg/kg avec un cycle d'administration d'au moins 1 semaine.

10. Composition pour utilisation selon la revendication 1, dans laquelle le polypeptide SPARC est destiné à être administré dans les 12 heures suivant l'administration du paclitaxel lié à de l'albumine, ou plus de 12 heures suivant l'administration du paclitaxel lié à de l'albumine.

11. Composition pour utilisation selon la revendication 1, dans laquelle le polypeptide SPARC et le paclitaxel lié à de l'albumine sont destinés à être administrés pratiquement simultanément.

12. Composition pour utilisation selon la revendication 1, dans laquelle le polypeptide SPARC et le paclitaxel lié à de l'albumine sont inclus dans une seule forme posologique.

13. Composition pour utilisation selon la revendication 1, dans laquelle le polypeptide SPARC et le paclitaxel lié à de l'albumine sont destinés à être administrés par voie intraveineuse.

14. Composition pour utilisation selon la revendication 1, dans laquelle la dose de paclitaxel lié à de l'albumine va de 30 mg/m² à 1000 mg/m² avec un cycle d'administration d'au moins 1 semaine, ou de 1 mg/m² à 30 mg/m² avec un cycle d'administration d'au moins 1 semaine, ou de 0,3 mg/m² à 1 mg/m² avec un cycle d'administration d'au moins 1 semaine, ou de 0,1 mg/m² à 0,3 mg/m² avec un cycle d'administration d'au moins 1 semaine.

15. Composition pour utilisation selon la revendication 1, laquelle composition réduit de 50 % le taux de croissance tumoral.

16. Composition pour utilisation selon la revendication 1, dans laquelle la tumeur de mammifère est choisie dans l'ensemble constitué par les tumeurs de la cavité orale, les tumeurs pharyngées, les tumeurs du système digestif, les tumeurs du système respiratoire, les tumeurs osseuses, les tumeurs cartilagineuses, les métastases osseuses, les sarcomes, les tumeurs cutanées, les mélanomes, les tumeurs mammaires, les tumeurs du système génital, les tumeurs des voies urinaires, les tumeurs orbitales, les tumeurs du cerveau et du système nerveux central, les gliomes, les tumeurs du système endocrinien, les tumeurs de la thyroïde, les tumeurs oesophagiennes, les tumeurs gastriques, les tumeurs de l'intestin grêle, les tumeurs du côlon, les tumeurs rectales, les tumeurs anales, les tumeurs du foie, les tumeurs de la vésicule biliaire, les tumeurs pancréatiques, les tumeurs laryngées, les tumeurs pulmonaires, les tumeurs bronchiques, le carcinome pulmonaire non à petites cellules, le carcinome pulmonaire à petites cellules, les tumeurs du col de l'utérus, les tumeurs du corps utérin, les tumeurs ovariennes, les tumeurs vulvaires, les tumeurs vaginales, les tumeurs de la prostate, le carcinome prostatique, les tumeurs testiculaires, les tumeurs du pénis, les tumeurs de la vessie, les tumeurs rénales, les tumeurs du bassinet du rein, les tumeurs de l'uretère, les tumeurs de la tête et du cou, le cancer parathyroïdien, la maladie de Hodgkin, le lymphome non hodgkinien, le myélome multiple, la leucémie, la leucémie aiguë lymphoblastique, la leucémie chronique lymphoblastique, la leucémie myéloïde aiguë, la leucémie myéloïde chronique et les tumeurs anales.

17. Composition pour utilisation selon la revendication 8, dans laquelle le polypeptide SPARC est destiné à être utilisé à une dose de 0,1 à 100 mg/kg par administration, avec un cycle d'administration d'au moins 1 semaine.

18. Composition pour utilisation selon la revendication 6, dans laquelle l'inhibiteur de l'angiogenèse est le sutent, l'avastin ou une de leurs combinaisons, et de préférence l'inhibiteur de l'angiogenèse est l'avastin.

19. Composition pour utilisation selon la revendication 18, dans lequel l'inhibiteur de l'angiogenèse est l'avastin et est destiné à être administré à une dose de 15 µg/kg à 15 mg/kg avec un cycle d'administration d'au moins 1 semaine.

20. Composition pour utilisation selon la revendication 6, dans laquelle l'inhibiteur de l'angiogenèse est le sutent et est destiné à être administré par voie orale.

21. Composition pour utilisation selon la revendication 1, dans laquelle l'inhibiteur de l'angiogenèse est destiné à être administré dans les 12 heures suivant l'administration du paclitaxel lié à de l'albumine, ou plus de 12 heures suivant l'administration du paclitaxel lié à de l'albumine.

22. Composition pour utilisation selon la revendication 1, dans laquelle l'inhibiteur de l'angiogenèse et le paclitaxel lié à de l'albumine, tel que le taxane, sont destinés à être administrés pratiquement simultanément.

23. Composition pour utilisation selon la revendication 1, dans laquelle le polypeptide SPARC, l'inhibiteur de l'angiogenèse et le paclitaxel lié à de l'albumine sont inclus dans une seule forme posologique.

24. Composition pour utilisation selon la revendication 1, dans laquelle le polypeptide SPARC, l'inhibiteur de l'angiogenèse et le paclitaxel lié à de l'albumine sont destinés à être administrés par voie intraveineuse.

25. Composition pour utilisation selon la revendication 1, dans laquelle la combinaison réduit le taux de croissance tumoral d'au moins 50 % en comparaison avec une monothérapie au paclitaxel lié à de l'albumine.

26. Composition pour utilisation selon la revendication 1, dans laquelle le polypeptide SPARC est à une dose de 40 µg/kg à 40 mg/kg par dose, l'inhibiteur de l'angiogenèse est l'avastin à une dose de 15 µg/kg à 15 mg/kg, et le paclitaxel lié à de l'albumine est à une dose de
(a) 30 mg/m² à 1000 mg/m² avec un cycle d'administration d'au moins 1 semaine,
(b) 1 mg/m³ à 30 mg/m² avec un cycle d'administration d'au moins 1 semaine,
(c) 0,3 mg/m² à 1 mg/m² avec un cycle d'administration d'au moins 1 semaine, ou
(d) 0,1 mg/m² à 0,3 mg/m² avec un cycle d'administration d'au moins 1 semaine.

27. Composition pour utilisation selon la revendication 26, dans laquelle le polypeptide SPARC comprend la SEQ ID NO : 1.

28. Composition pour utilisation selon la revendication 26, dans laquelle le polypeptide SPARC comprend la SEQ ID NO : 3.

29. Composition pour utilisation selon la revendication 1, dans laquelle le polypeptide SPARC est lyophilisé.

30. Composition pour utilisation selon la revendication 1, dans laquelle le paclitaxel lié à de l'albumine est lyophilisé.

31. Composition pour utilisation selon la revendication 6, dans laquelle l'inhibiteur de l'angiogenèse est l'avastin et dans laquelle l'avastin est lyophilisé.
